# EUROPEAN PATENT APPLICATION

(11) **EP 2 845 585 A1**
(43) Date of publication of application: **11.03.2015**
(21) Application number: 13758372.0
(22) Date of filing: 05.03.2013
(51) Int. Cl.: A61K 8/60, A61Q 1/02, A61Q 1/06, A61Q 1/10, A61Q 1/12, A61Q 1/14, A61Q 5/06, A61Q 19/00, C09K 3/00, C07H 13/06

(54) **TREHALOSE FATTY ACID ESTER COMPOSITION**

(30) Priority: 06.03.2012 US 201261607177 P
(71) Applicant: The Nisshin Oillio Group, Ltd., Chuo-ku Tokyo 104-8285 (JP)
(72) Inventor: MORIKAKU Yuuhei, Yokohama-shi Kanagawa 235-8558 (JP); KOBAYASHI Tatsuya, Yokohama-shi Kanagawa 235-8558 (JP); SHIBATA Masashi, Hachioji-shi Tokyo 192-0982 (JP)
(74) Representative: Cabinet Laurent & Charras
(86) International application number: PCT/JP2013/055993
(87) International publication number: WO 2013/133271

(57) **Abstract**

The present invention relates to a trehalose fatty acid ester composition, including a trehalose fatty acid ester, wherein all the fatty acid residues held by all the trehalose fatty acid esters in the composition are saturated fatty acid residues having 8 to 22 carbon atoms, the composition contains at least two types of esters selected from the group consisting of a triester, a tetraester, a pentaester, a hexaester, a heptaester, and an octaester, and the sum amount of the esters relative to the total amount of the trehalose fatty acid ester in the composition, is from 20 to 100% by area. According to the present invention, it is possible to provide a composition which has excellent thermostability and an excellent effect of adjusting the hardness for various types of waxes, which can be used as a solidifier together with a wax for various types of cosmetics, and which can add excellent shape retainability, favorable sense of use, and make-up lasting, to the cosmetic blended with the composition.

## Description

### TECHNICAL FIELD

The present invention relates to a trehalose fatty acid ester composition which excels in adjusting the hardness of a cosmetic or the like, and a cosmetic including the composition.

Priority is claimed on United States Patent Application No. 61/607,177, filed March 6, 2012 in the United States, the content of which is incorporated herein by reference.

### BACKGROUND ART

Until now, various types, applications, or formulations of solid form cosmetics have been developed. Their formulations are various over wide ranges, for example, such as a cosmetic for lips like a rouge and a lip cream having been formed in a stick shape and filled in a cylindrical applicator for dispensing by rolling, an oil type foundation formed by pouring in a metal tray, or a pencil type eyebrow pencil in which the cosmetic itself is enclosed by a wood material like a pencil. These solid form cosmetics have various formulations, although they have a common point in that their respective formulations are kept by being blended with a component such as a wax or a synthetic resin, which are solid at normal temperatures.

One of the most important factors for forming the shapes of these solid form cosmetics can be given by the crystallinity control of these components which are solid at normal temperatures. In general, it is known that the formulation and other properties of the solid form cosmetic are largely changed by the size of the crystals (crystal diameter) and the growth rate of the crystals. For example, a paraffin wax which is used for many applications as a wax is known to be changed in the crystal diameter due to the cooling rate. It is also known that a solidified product formed of coarse crystals has a much more inferior hardness as a whole of the solidified product, than that of a solidified product formed of fine crystals.

A substance called a crystal modifier is used to control the crystal diameter, in other words, to control the quality of the final formulation. It is general to use and blend a crystal modifier in combination with a component such as a wax or a synthetic resin, which are solid at normal temperatures, so as to achieve the targeted formulation. As to the crystal modifier, usually used is a component which is different in the dissolubility, the melting point, the crystal size per se, or the like, from the major component which is solid at normal temperatures. The component is usually blended at an amount to serve as an additive for the major component.

One of the problems for using the crystal modifier is that the addition amount to be blended should be precise. As described above, the crystal modifier is different in various physical properties from the major component. Thus, unless a certain amount has been added, the crystal modification effect may not be exerted; while, conversely, if too much amount has been added, the targeted quality of the major component may be impaired.

Regarding this problem, a specific example can be given by a rouge as one of the cosmetics for lips.

The components of a rouge can be largely classified into: a pigment or a dye for giving a vivid color to the lips; a wax which is solid at normal temperatures as an excipient component; or an oil solution for evenly dispersing or dissolving these components, and also adding functions such as the sense of use, the glossiness, and the like. In the cosmetic for a rouge, the shape retainability and the sense of use (particularly, the smooth spreading ability at the time of the application, or the glossiness) required for the product should be satisfied by adjusting the blending proportion of the wax and the liquid oil.

However, it is very difficult to satisfying both the sufficient shape retainability and the sufficient sense of use. Since the wax is an essential component for solidifying the liquid oil, a sufficient amount should be blended so as to retain the shape of the product at the time of use (the shape retainability). On the other hand, if the blending amount of the wax is large, the hardness of the rouge as a whole is too much. As a result, the sense of use at the time of the application may be significantly lowered, the glossiness achieved by the liquid oil may be lowered, and furthermore, the colorability which is the original purpose of the rouge may be lowered. Conversely, if the addition amount of the wax is reduced so as to satisfy the sense of use, the sense of use is improved because the hardness to the lips is lowered. However, the rouge might be broken at the time of use because the necessary hardness as a rouge can not be kept, or the rouge might be melt because the formulation can not be kept when carried in summer.

In this way, a cosmetic for a rouge which excels in both the shape retainability and the sense of use is a long time demand in the market, and attempts for the development have made by many researchers.

In recent years, in order to achieve both the shape retainability and the sense of use, it is usual to use an ester based wax which is synthesized by binding two or more fatty acids to poly alcohol so as to have a branched structure, in combination with a wax component having a linear structure and relatively high melting point, such as a polyethylene wax or a paraffin wax. The ester based wax is used as a crystal modifier for a polyethylene wax or a paraffin wax as the major component. By using the ester based wax in combination, the crystal structure is densified and the hardness as a solid form cosmetic is increased. As a result, it becomes possible to reduce the wax content in the whole system, and therefore, the sense of use can be satisfied. Moreover, it is known to be possible, by using two more waxes having different physical properties in combination, to make a precise difference in the sense of use, and to dramatically improve the thermal resistance and the like of the formulation.

One of the ester based waxes for general use as a crystal modifier can be exemplified by a sucrose fatty acid ester based wax. For example, Patent Document 1 describes an example in which a sucrose fatty acid esters is applied to a stick form cosmetic. Moreover, Patent Document 2 describes that a modifier containing a sucrose saturated fatty acid ester having a specific esterification degree, among sucrose saturated fatty acid esters, at a specific weight ratio, has a high crystallizing inhibitory effect for solid oil and fat.

In addition, Patent Document 3 describes that a trehalose fatty acid ester including a specific composition has excellent stability for dispersing a pigment, and it is possible, by blending the trehalose fatty acid ester, to produce a cosmetic having a larger amount of blended pigments, and being excellent in the sense of use, the make-up lasting, the odor, and the stability over time. Furthermore, it is described that the trehalose fatty acid ester has an effect of enhancing the hardness, and thus brings a higher effect of reinforcing the shape by enhancing the hardness when used in combination with waxes, rather than the case of using a general oil solution.

### PRIOR ART DOCUMENTS

### PATENT DOCUMENTS

Patent Document 1: Japanese Examined Patent Application, Second Publication No. S53-006219
Patent Document 2: Japanese Unexamined Patent Application, First Publication No. 2004-131524
Patent Document 3: PCT International Publication No. WO 2007/063902 pamphlet

### DISCLOSURE OF INVENTION

### PROBLEMS TO BE SOLVED BY THE INVENTION

The sucrose fatty acid ester has insufficient thermostability, and may deteriorate the quality of the cosmetic added with the ester by heating at the time of the production. Moreover, it has been difficult to determine an appropriate blending amount as a crystal modifier.

The present invention provides a composition which has excellent thermostability and an excellent effect of adjusting the hardness for various types of waxes, which can be used as a solidifier together with a wax for various types of cosmetics, and which can add excellent shape retainability, favorable sense of use, and make-up lasting, to the cosmetic blended with the composition.

### MEANS TO SOLVE THE PROBLEMS

The inventors of the present invention have conducted earnest studies to solve the above-mentioned problems, and as a result, they have found that: a composition including a trehalose fatty acid ester having a specific fatty acid residue and a specific hydroxyl value, is able to adjust the hardness of a wax composition obtained by blending in a wax; the fluctuation of the hardness of the wax composition along with the variation of the blending amount is moderate; and furthermore, the thermostability is excellent. This has led to the completion of the present invention.

The trehalose fatty acid ester composition, the wax composition, the crystal modifier, and the solid cosmetic of the present invention have the following aspects [1] to [13], for example.
[1] A trehalose fatty acid ester composition, including a trehalose fatty acid ester of trehalose and a fatty acid, wherein:
   over 50% of all the fatty acid residues in the trehalose fatty acid ester are one or more types of residues selected from the group consisting of a palmitic acid residue and a linear stearic acid residue, and
   over 90% of all the fatty acid residues in the trehalose fatty acid ester are a palmitic acid residue, and
   the hydroxyl value is from 15 to 110; or
   over 60% of all the fatty acid residues in the trehalose fatty acid ester are a linear stearic acid residue, and
   the hydroxyl value is from 15 to 110.
[2] The trehalose fatty acid ester composition according to [1], wherein:
   (1) over 90% of all the fatty acid residues in the trehalose fatty acid ester are a palmitic acid residue, and
      the hydroxyl value is from 15 to 45;
   (2) over 90% of all the fatty acid residues in the trehalose fatty acid ester are a linear stearic acid residue, and
      the hydroxyl value is from 15 to 110; or
   (3) over 90% of all the fatty acid residues in the trehalose fatty acid ester are one or more types of residues selected from the group consisting of a palmitic acid residue and a linear stearic acid residue,
   over 50% of all the fatty acid residues are a linear stearic acid residue, and the hydroxyl value is from 15 to 110.
[3] A wax composition, including the trehalose fatty acid ester composition according to either one of [1] and [2].
[4] A crystal modifier, including the trehalose fatty acid ester composition according to either one of [1] and [2].
[5] The crystal modifier according to [4], further including a wax.
[6] The crystal modifier according to either one of [4] and [5], for use in a solid cosmetic.
[7] A solid cosmetic, including the trehalose fatty acid ester composition according to either one of [1] and [2].
[8] The solid cosmetic according to [7], including the trehalose fatty acid ester composition at 4 to 20% by mass.
[9] The solid cosmetic according to [8], further including a wax.
[10] The solid cosmetic according to [9], wherein the sum amount of the trehalose fatty acid ester composition and the wax is from 20 to 30% by mass.
[11] The solid cosmetic, including the wax composition according to either one of [3] and [4].
[12] The solid cosmetic according to [11], wherein the amount of the wax composition is from 15 to 30% by mass.
[13] The solid cosmetic, including the crystal modifier according to any one of [4] to [6].

Moreover, the present invention has the following aspects.
[1] A trehalose fatty acid ester composition, including a trehalose fatty acid ester, wherein
   all the fatty acid residues held by all the trehalose fatty acid esters in the composition are linear saturated fatty acid residues having 8 to 22 carbon atoms,
   the composition contains at least two types of esters selected from the group consisting of a triester, a tetraester, a pentaester, a hexaester, a heptaester, and an octaester, and
   the sum amount of the esters relative to the total peak area, calculated by high performance liquid chromatography analysis is from 20 to 100% by area.
[2] The trehalose fatty acid ester composition according to [1], wherein
   the composition contains at least two types of esters selected from the group consisting of a pentaester, a hexaester, a heptaester, and an octaester, and
   the sum amount of the esters relative to the total peak area, calculated by high performance liquid chromatography analysis is from 30 to 100% by area.
[3] The trehalose fatty acid ester composition according to either one of [1] and [2], wherein:
   over 90% by mass of all the fatty acid residues held by all the trehalose fatty acid esters in the composition are a palmitic acid residue,
   the hydroxyl value of the trehalose fatty acid ester is from 15 to 110, and
   the sum amount of a pentaester, a hexaester, a heptaester, and an octaester, relative to the total peak area, calculated by high performance liquid chromatography analysis is from 80 to 100% by area; or
   over 60% by mass of all the fatty acid residues held by all the trehalose fatty acid esters in the composition are a stearic acid residue,
   the hydroxyl value is from 15 to 110, and
   the sum amount of a pentaester, a hexaester, a heptaester, and an octaester, relative to the total peak area, calculated by high performance liquid chromatography analysis is from 70 to 100% by area.
[4] The trehalose fatty acid ester composition according to either one of [1] and [2], wherein:
   (1) over 90% by mass of all the fatty acid residues held by all the trehalose fatty acid esters in the composition are a palmitic acid residue,
      the hydroxyl value is from 15 to 45,
      the sum amount of a hexaester, relative to the total peak area, calculated by high performance liquid chromatography analysis is from 10 to 25% by area, and
      the sum amount of a heptaester and an octaester, relative to the total peak area, calculated by high performance liquid chromatography analysis is from 70 to 90% by area;
   (2) over 90% by mass of all the fatty acid residues held by all the trehalose fatty acid esters in the composition are a stearic acid residue,
      the hydroxyl value is from 15 to 110,
      the sum amount of a tetraester, a pentaester, and a hexaester, relative to the total peak area, calculated by high performance liquid chromatography analysis is from 10 to 70% by area, and
      the sum amount of a heptaester and an octaester, relative to the total peak area, calculated by high performance liquid chromatography analysis is from 20 to 90% by area; or
   (3) over 90% by mass of all the fatty acid residues held by all the trehalose fatty acid esters in the composition are at least one type of residue selected from the group consisting of a palmitic acid residue and a stearic acid residue,
   all the trehalose fatty acid esters in the composition have both the palmitic acid residue and the stearic acid residue,
   over 50% by mass of all the fatty acid residues are a stearic acid residue,
   the hydroxyl value is from 15 to 110,
   the sum amount of a tetraester, a pentaester, and a hexaester, relative to the total peak area, calculated by high performance liquid chromatography analysis is from 22 to 70% by area, and
   the sum amount of a heptaester and an octaester, relative to the total peak area, calculated by high performance liquid chromatography analysis is from 20 to 90% by area.
[5] A wax composition, including the trehalose fatty acid ester composition according to any one of [1] to [4].
[6] A crystal modifier, including the trehalose fatty acid ester composition according to any one of [1] to [4].
[7] The crystal modifier according to [6], further including a wax.
[8] The crystal modifier according to either one of [6] and [7], for use in a solid cosmetic.
[9] A solid cosmetic, including the trehalose fatty acid ester composition according to any one of [1] to [4].
[10] The solid cosmetic according to [9], including the trehalose fatty acid ester composition at 0.5 to 30% by mass relative to the total mass of the solid cosmetic.
[11] The solid cosmetic according to [10], further including a wax.
[12] The solid cosmetic according to [11], wherein the sum amount of the trehalose fatty acid ester composition and the wax relative to the total mass of the solid cosmetic is from 1 to 30% by mass.
[13] A solid cosmetic, including the wax composition according to [5].
[14] The solid cosmetic according to [13], wherein the amount of the wax composition relative to the total mass of the solid cosmetic is from 1 to 30% by mass.
[15] A solid cosmetic, including the crystal modifier according to any one of [6] to [8].
   Furthermore, the present invention has the following other aspects.

«1» A trehalose fatty acid ester composition, including a trehalose fatty acid ester, wherein
   all the fatty acid residues held by all the trehalose fatty acid esters in the composition are linear saturated fatty acid residues having 8 to 22 carbon atoms,
   the hydroxyl value of the trehalose fatty acid ester is from 15 to 110, and
   the sum amount of a pentaester, a hexaester, a heptaester, and an octaester, relative to the total peak area, calculated by high performance liquid chromatography analysis is from 70 to 100% by area.
«2» The trehalose fatty acid ester composition according to «1», wherein:
   over 90% by mass of all the fatty acid residues held by all the trehalose fatty acid esters in the composition are a palmitic acid residue,
   the hydroxyl value of the trehalose fatty acid ester is from 15 to 45,
   the sum amount of a hexaester and a heptaester, relative to the total peak area, calculated by high performance liquid chromatography analysis is from 50% by area; or
   over 60% by mass of all the fatty acid residues held by all the trehalose fatty acid esters in the composition are a stearic acid residue;
   the hydroxyl value is from 15 to 110, and
   the sum amount of a pentaester, a hexaester, and a heptaester, relative to the total peak area, calculated by high performance liquid chromatography analysis is 30% by area or more.
«3» The trehalose fatty acid ester composition according to «1», wherein:
   (1) over 90% by mass of all the fatty acid residues held by all the trehalose fatty acid esters in the composition are a palmitic acid residue,
      the hydroxyl value is from 15 to 45, and
      the sum amount of a hexaester and a heptaester, relative to the total peak area, calculated by high performance liquid chromatography analysis is from 50 to 70% by area;
   (2) over 90% by mass of all the fatty acid residues held by all the trehalose fatty acid esters in the composition are a stearic acid residue,

      the hydroxyl value is from 15 to 110, and
      the sum amount of a pentaester, a hexaester, and a heptaester, relative to the total peak area, calculated by high performance liquid chromatography analysis is from 30 to 80% by area; or
   (3) over 90% by mass of all the fatty acid residues held by all the trehalose fatty acid esters in the composition are at least one type of residue selected from the group consisting of a palmitic acid residue and a stearic acid residue,
      all the trehalose fatty acid esters in the composition have both the palmitic acid residue and the stearic acid residue,
      over 50% by mass of all the fatty acid residues are a stearic acid residue,
      the hydroxyl value is from 15 to 110, and
      the sum amount of a hexaester and a heptaester, relative to the total peak area, calculated by high performance liquid chromatography analysis is from 30 to 80% by area.
«4» A wax composition, including the trehalose fatty acid ester composition according to any one of «1» to «3».
«5» A crystal modifier, including the trehalose fatty acid ester composition according to any one of «1» to «3».
«6» A crystal modifier according to «5», further including a wax.
«7» A crystal modifier according to either one of «5» and «6», for use in a solid cosmetic.
«8» A solid cosmetic, including the trehalose fatty acid ester composition according to any one of «1» to «3».
«9» The solid cosmetic according to «8», including the trehalose fatty acid ester composition at 0.5 to 30% by mass relative to the total mass of the solid cosmetic. «10» The solid cosmetic according to «9», further including a wax.
«11» The solid cosmetic according to «10», wherein the sum amount of the trehalose fatty acid ester composition and the wax relative to the total mass of the solid cosmetic is from 1 to 30% by mass.
«12» A solid cosmetic, including the wax composition according to «4».
«13» The solid cosmetic according to «12», wherein the amount of the wax composition relative to the total mass of the solid cosmetic is from 1 to 30% by mass.
«14» A solid cosmetic, including the crystal modifier according to any one of «5» to «7».

### EFFECT OF THE INVENTION

The trehalose fatty acid ester composition of the present invention is able to exert a moderate hardening effect dependently on the blending amount, when added to a wax. Even though the composition is blended at a high rate relative to a wax, an excellent excipient function is seen. Furthermore, the composition also excels in the thermostability at the time of the production. For this reason, the trehalose fatty acid ester composition of the present invention is suitable as a crystal modifier to be blended in a wax, and can be suitably used for a solid cosmetic which is required to be excellent in both the shape retainability and the sense of use.

### BRIEF DESCRIPTION OF THE DRAWINGS

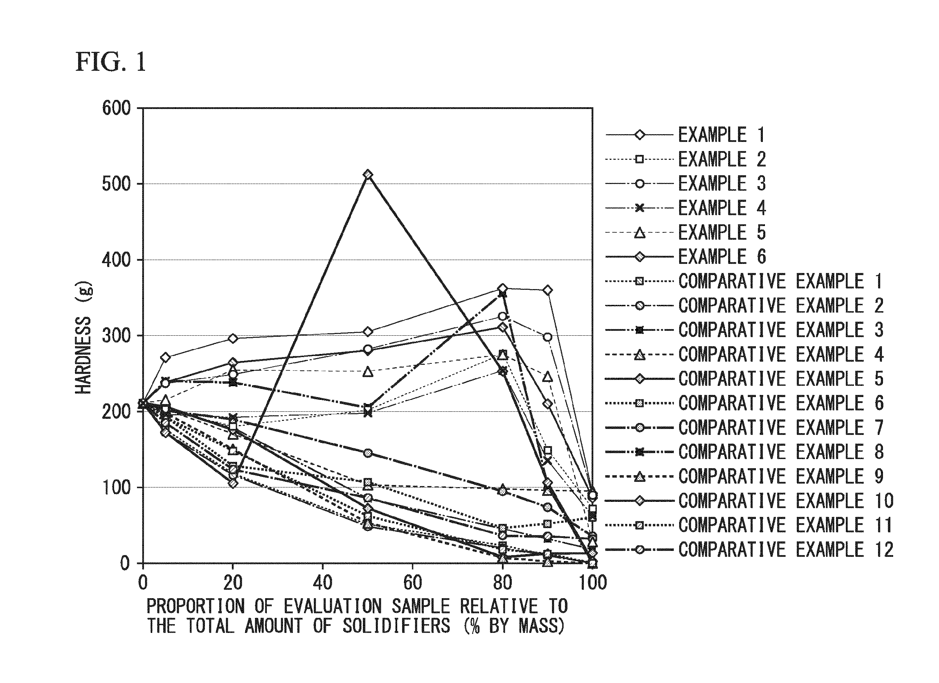

FIG. 1 is a graph showing the measurement results of the hardness of respective mixtures of Examples 1 to 6 and Comparative Examples 1 to 12.

### EMBODIMENTS FOR CARRYING OUT THE INVENTION

In the present invention and the description of this application, the hydroxyl value refers to a value obtained by the Hydroxyl Value Determination in General Tests in the Standards of Cosmetic Ingredients. Moreover, the average esterification degree can be calculated from the hydroxyl value obtained by this determination method and the theoretical hydroxyl value of respective esters.

Unless otherwise specifically stated, the amount of each trehalose fatty acid ester refers to an area percentage (% by area) as measured by high performance liquid chromatography analysis (hereinafter, abbreviated as HPLC). The measurement by HPLC can be carried out by using a differential refraction index (RI) method with reference to "Determination of Sucrose Fatty Acid Ester by High-performance Liquid Chromatography; J. Oleo Sci., Vol. 50, No. 4 (2001)". For the analysis of each ester in the trehalose fatty acid ester composition, all of the esters can not be separated under only one measurement condition, and thus, a combination of two measurement conditions using GPC columns and ODS columns can be used to analyze all of the esters. Moreover, since the differential refraction index (RI) method is an analysis method based on the difference in the refraction index of a solution, the size of the peak serves as the ratio of the amount of each component. For this reason, the % by area measured by the differential refraction index (RI) can be deemed as the % by mass of each component in the composition.

The % by area of the remaining raw materials, a monoester, a diester, a triester, and a tetraester can be calculated under the measurement condition using GPC columns. Since a pentaester, a hexaester, a heptaester, and an octaester cannot be separated from each other under the measurement condition using GPC columns, the measurement can be done by using the value as a polyester (a mixture of a pentaester, a hexaester, a heptaester, and an octaester). Since a pentaester, a hexaester, a heptaester, and an octaester can be separated from each other under the measurement condition using ODS columns, the % by area of a pentaester, a hexaester, a heptaester, and an octaester can be calculated from the ratio of a pentaester, a hexaester, a heptaester, and an octaester in the polyester as calculated under the measurement condition using ODS columns, and from the % by area of the polyester as calculated under the measurement condition using GPC columns. The analysis methods (measurement conditions) and the calculation methods in this case are described below in detail.

### [Measurement Conditions of High Performance Liquid Chromatography Analysis]

The measurement condition of high performance liquid chromatography analysis to calculate the % by area of a monoester, a diester, a triester, a tetraester, and a polyester in the trehalose fatty acid ester composition (Measurement Condition A) is as follows. The term polyester refers to a mixture of a pentaester, a hexaester, a heptaester, and an octaester.
Column: Four styrene divinylbenzene-based GPC columns, connected in a series, each being 7.8 mm in inner diameter; 300 mm in length, and 5 µm in size
Mobile phase: Tetrahydrofuran
Column temperature: 40°C
Flow rate of mobile phase: 0.5 mL/min
Detection: Differential refraction index (RI)

The measurement condition of high performance liquid chromatography analysis to calculate the ratio of a pentaester, a hexaester, a heptaester, and an octaester in the polyester in the trehalose fatty acid ester composition (Measurement Condition B) is as follows.
Column: Two ODS columns, connected in a series, each being 4.6 mm in inner diameter; 150 mm in length, and 5 µm in size
Mobile phase: Tetrahydrofuran:Methanol = 50:50 (volume ratio)
Column temperature: 40°C
Flow rate of mobile phase: 1.0 mL/min
Detection: Differential refraction index (RI)

### [Method for calculating Area Percentage (% by Area) of Each Ester]

The method for calculating the % by area of a monoester, a diester, a trimester, and a tetraester is as follows (Calculation Method (1)).

The percentage of each peak area of raw materials, a monoester, a diester, a triester, and a tetraester, relative to the total peak area, obtained by the measurement by means of high performance liquid chromatography analysis using the GPC columns under the measurement condition A, is taken as the % by area of each ester.

The method for calculating the % by area of a polyester is as follows (Calculation Method (2)).

The percentage (X) of the sum peak area of the components other than the raw materials, the monoester, the diester, the triester, and the tetraester, relative to the total peak area, obtained by the measurement by means of high performance liquid chromatography analysis using the GPC columns under the measurement condition A, is taken as the % by area of a polyester.

The method for calculating the ratio of a pentaester, a hexaester, a heptaester, and an octaester in the polyester is as follows (Calculation Method (3)).

The sum peak area of a pentaester, a hexaester, a heptaester, and an octaester, obtained by the measurement by means of high performance liquid chromatography analysis using the ODS column under the measurement condition B, is taken as (Y), and the ratio of each peak area of the pentaester, the hexaester, the heptaester, and the octaester, relative to (Y) is respectively calculated and taken as the ratio of the pentaester, the hexaester, the heptaester, and the octaester in the polyester.

The method for calculating the % by area of a pentaester, a hexaester, a heptaester, and an octaester is as follows (Calculation Method (4)).

The value obtained by respectively multiplying the % by area (X) of the polyester as calculated in the calculation method (2) with the ratio of each peak area of the pentaester, the hexaester, the heptaester, and the octaester in the polyester as calculated in the calculation method (3), is taken as the % by area of the pentaester, the hexaester, the heptaester, and the octaester.

The method for calculating the sum amount of respective esters is as follows (Calculation Method (5)).

The % by area obtained from the sum of the % by area of respective esters as calculated in the calculation method (1) or the calculation method (4) is taken as the sum amount of respective esters.

For example, the sum amount of a diester, a triester, a tetraester, and a pentaester can be calculated by adding the % by area of the diester, the trimester, and the tetraester as calculated in the calculation method (1), to the % by area of the pentaester as calculated in the calculation method (4).

### <Trehalose fatty acid ester composition>

The trehalose fatty acid ester composition of the present invention is a composition including a trehalose fatty acid ester, wherein all the fatty acid residues held by all the trehalose fatty acid esters in the composition are fatty acid residues having 8 to 22 carbon atoms, at least two types of esters selected from the group consisting of a triester, a tetraester, a pentaester, a hexaester, a heptaester, and an octaester, are contained, and the sum amount of the esters relative to the total peak area, calculated by HPLC analysis of the trehalose fatty acid ester in the composition is from 20 to 100% by area. The composition is able to exert an excellent hardening effect when added to a wax, by increasing the proportion of linear saturated fatty acid residues in all the fatty acid residues of all the trehalose fatty acid esters in the composition higher than that of branched saturated fatty acid residues, as well as increasing the ratio of the amount of a tetraester, a pentaester, a hexaester, and a heptaester, within the trehalose fatty acid esters to be sufficiently high.

In the present invention and the description of this application, the trehalose fatty acid ester refers to an ester in which at least a part of the hydroxyl groups of trehalose are substituted with fatty acid residues. In the trehalose fatty acid ester composition of the present invention, over 50% by mass of all the fatty acid residues held by all the contained trehalose fatty acid esters are substituted with linear saturated fatty acid residues having 8 to 22 carbon atoms. In the trehalose fatty acid ester composition of the present invention, the proportion of linear saturated fatty acid residues having 8 to 22 carbon atoms relative to all the fatty acid residues held by all the contained trehalose fatty acid esters is preferably 70% by mass or more and 100% by mass or less, more preferably 80% by mass or more and 100% by mass or less, and yet more preferably 90% by mass or more and 100% by mass or less.

The linear saturated fatty acid residue is not specifically limited as long as it is a linear saturated fatty acid residue having 8 to 22 carbon atoms. Examples thereof can include a caprylic acid (octanoic acid) residue, a capric acid (decanoic acid) residue, a lauric acid (dodecanoic acid) residue, a myristic acid (tetradecanoic acid) residue, a palmitic acid (hexadecanoic acid) residue, a stearic acid (octadecanoic acid) residue, or a behenic acid (docosanoic acid) residue. It is either possible to use a single type, or a combination of two or more types, of the linear saturated fatty acid residues having 8 to 22 carbon atoms, in the trehalose fatty acid esters contained in the trehalose fatty acid ester composition of the present invention (hereunder, may be referred to as "the trehalose fatty acid ester of the present invention"). The linear saturated fatty acid residue in the trehalose fatty acid ester of the present invention is preferably a palmitic acid residue or a stearic acid residue.

It is preferable that the trehalose fatty acid ester of the present invention includes at least one of a palmitic acid residue and a stearic acid residue, as the fatty acid residue. Moreover, it is preferable that over 50% by mass and 100% by mass or less relative to all the fatty acid residues of all the trehalose fatty acid esters in the trehalose fatty acid ester composition of the present invention are at least one type of residue selected from the group consisting of a palmitic acid residue and a stearic acid residue, and it is more preferable that over 60% by mass and 100% by mass or less of all the fatty acid residues are at least one type of residue selected from the group consisting of a palmitic acid residue and a stearic acid residue.

Unless otherwise stated, the proportion of a fatty acid residue in the present invention refers to the % by mass as determined by fatty acid composition analysis using gas chromatography (hereunder, abbreviated as FA composition analysis).

It is preferable that the trehalose fatty acid ester composition of the present invention contains at least two types of esters having different esterification degrees. The trehalose fatty acid ester composition of the present invention is preferably such that the sum amount of at least two types of esters selected from the group consisting of a triester, a tetraester, a pentaester, a hexaester, a heptaester, and an octaester, relative to the total peak area calculated by HPLC analysis, is from 20 to 100% by area, more preferably from 70 to 100% by area, yet more preferably from 80 to 100% by area, and most preferably from 95 to 100% by area.

Moreover, the average esterification degree of the trehalose fatty acid ester of the present invention is preferably from 5 to 8, and more preferably from 5 to 7.

In cases where all the fatty acid residues held by all the trehalose fatty acid esters in the trehalose fatty acid ester composition of the present invention are linear saturated fatty acid residues having 8 to 22 carbon atoms, and at least two types of esters selected from the group consisting of a pentaester, a hexaester, a heptaester, and an octaester, are contained; it is preferable that the sum amount of the esters relative to the total peak area calculated by HPLC analysis is from 30 to 100% by area, more preferably from 60 to 100% by area, and yet more preferably from 70 to 100% by area.

In cases where over 90% by mass and 100% by mass or less relative to all the fatty acid residues held by all the trehalose fatty acid esters in the trehalose fatty acid ester composition of the present invention are a palmitic acid residue, the hydroxyl value of the trehalose fatty acid ester is preferably 15 or more, and more preferably 20 or more. Moreover, the hydroxyl value is preferably 110 or less, more preferably 70 or less, and yet more preferably 45 or less. The range of the hydroxyl value is preferably from 15 to 110, more preferably from 15 to 70, yet more preferably from 15 to 45, and particularly preferably from 20 to 25.

Furthermore, the average esterification degree of the trehalose fatty acid ester is preferably from 6 to 7.

In cases where over 90% by mass and 100% by mass or less relative to all the fatty acid residues held by all the trehalose fatty acid esters in the trehalose fatty acid ester composition of the present invention are a palmitic acid residue, the trehalose fatty acid ester composition of the present invention is preferably such that the sum amount of a pentaester, a hexaester, a heptaester, and an octaester, relative to the total peak area, calculated by HPLC analysis, is from 80 to 100% by area, and more preferably from 90 to 100% by area.

Moreover, it is preferable that the sum amount of a hexaester, relative to the total peak area, calculated by HPLC analysis, is from 10 to 25% by area, more preferably from 13 to 23% by area, and yet more preferably from 17 to 22% by area. In addition, it is preferable that the sum amount of a heptaester and an octaester, relative to the total peak area, calculated by HPLC analysis, is from 70 to 90% by area, more preferably from 70 to 85% by area, and yet more preferably from 70 to 80% by area.

In cases where over 60% by mass and 100% by mass or less relative to all the fatty acid residues held by all the trehalose fatty acid esters in the trehalose fatty acid ester composition of the present invention are a stearic acid residue, the hydroxyl value of the trehalose fatty acid ester is preferably 15 or more, and more preferably 20 or more. Moreover, the hydroxyl value is preferably 110 or less, more preferably 100 or less, and yet more preferably 50 or less. The range of the hydroxyl value is preferably from 15 to 110, more preferably from 20 to 100, yet more preferably from 20 to 50, and most preferably from 28 to 40.

Furthermore, the average esterification degree of the trehalose fatty acid ester is preferably from 5 to 7.5.

In cases where over 60% by mass and 100% by mass or less relative to all the fatty acid residues held by all the trehalose fatty acid esters in the trehalose fatty acid ester composition of the present invention are a stearic acid residue, the trehalose fatty acid ester composition of the present invention is preferably such that the sum amount of a pentaester, a hexaester, a heptaester, and an octaester, relative to the total peak area, calculated by HPLC analysis, is from 70 to 100% by area, more preferably from 80 to 100% by area, yet more preferably from 90 to 100% by area, and most preferably from 95 to 100% by area.

In cases where over 90% by mass and 100% by mass or less relative to all the fatty acid residues held by all the trehalose fatty acid esters in the trehalose fatty acid ester composition of the present invention are a stearic acid residue, the hydroxyl value of the trehalose fatty acid ester is preferably from 15 to 110, more preferably from 20 to 100, yet more preferably from 30 to 100, and most preferably from 35 to 94.

Moreover, the average esterification degree of the trehalose fatty acid ester is preferably from 5 to 7.

In cases where over 90% by mass and 100% by mass or less relative to all the fatty acid residues held by all the trehalose fatty acid esters in the trehalose fatty acid ester composition of the present invention are a stearic acid residue, the trehalose fatty acid ester composition of the present invention is preferably such that the sum amount of a tetraester, a pentaester, and a hexaester, relative to the total peak area, calculated by HPLC analysis, is from 10 to 70% by area, more preferably from 20 to 70% by area, and yet more preferably from 37 to 64% by area. Moreover, it is preferable that the sum amount of a heptaester and an octaester, relative to the total peak area, calculated by HPLC analysis, is from 20 to 90% by area, more preferably from 20 to 80% by area, yet more preferably from 20 to 70% by area, and most preferably from 25 to 60% by area.

In cases where over 90% by mass and 100% by mass or less of all the fatty acid residues held by all the trehalose fatty acid esters in the trehalose fatty acid ester composition of the present invention are at least one type of residue selected from the group consisting of a palmitic acid residue and a stearic acid residue, and all the trehalose fatty acid esters in the trehalose fatty acid ester composition contain both the palmitic acid residue and the stearic acid residue; the hydroxyl value of the trehalose fatty acid ester is preferably from 15 to 110, more preferably from 15 to 105, yet more preferably from 20 to 100, and most preferably from 28 to 99. Moreover, the average esterification degree of the trehalose fatty acid ester is preferably from 5 to 7. The mass ratio of the palmitic acid residue to the stearic acid residue is preferably from 3:7 to 1:9, and more preferably 1:9.

In cases where over 90% by mass and 100% by mass or less of all the fatty acid residues held by all the trehalose fatty acid esters in the trehalose fatty acid ester composition of the present invention are at least one type of residue selected from the group consisting of a palmitic acid residue and a stearic acid residue, all the trehalose fatty acid esters in the trehalose fatty acid ester composition contain both the palmitic acid residue and the stearic acid residue, and over 50% by mass and 100% by mass or less of all the fatty acid residues are a stearic acid residue, the hydroxyl value of the trehalose fatty acid ester is preferably from 15 to 110, more preferably from 15 to 105, yet more preferably from 20 to 100, and most preferably from 28 to 99.

In cases where over 90% by mass and 100% by mass or less relative to all the fatty acid residues held by all the trehalose fatty acid esters in the trehalose fatty acid ester composition of the present invention are at least one type of residue selected from the group consisting of a palmitic acid residue and a stearic acid residue, all the trehalose fatty acid esters in the trehalose fatty acid ester composition contain both the palmitic acid residue and the stearic acid residue, and over 50% by mass and 100% by mass or less of all the fatty acid residues are a stearic acid residue; it is preferable that the sum amount of a tetraester, a pentaester, and a hexaester, relative to the total peak area, calculated by HPLC analysis, is from 22 to 70% by area, more preferably from 22 to 65% by area, and yet more preferably from 24 to 65% by area. Moreover, it is preferable that the sum amount of a heptaester and an octaester, relative to the total peak area, calculated by HPLC analysis, is from 20 to 90% by area, more preferably from 20 to 85% by area, yet more preferably from 20 to 80% by area, and most preferably from 24 to 76% by area.

The method for synthesizing the trehalose fatty acid ester of the present invention is not specifically limited, and it is possible to synthesize by a usual method for synthesizing an ester of a sugar and a fatty acid, for example. Specifically speaking, it is possible to synthesize an ester by directly reacting trehalose with a fatty acid, or to synthesize an ester by reacting trehalose with a fatty acid ester. In addition, some refined fatty acids available in the market contain other fatty acids as impurities, and such fatty acids may also be used as a raw material for the synthesis.

The form of the trehalose fatty acid ester composition of the present invention is not specifically limited, and can be appropriately determined according to the type and the amount of the contained trehalose fatty acid ester of the present invention. The form may be a solid form, or may be a paste form.

The trehalose fatty acid ester composition of the present invention may also contain another component as long as the effect for adjusting the hardness of the wax by the trehalose fatty acid ester of the present invention would not be impaired. Specifically, such another component can be exemplified by a surfactant such as a nonionic surfactant, an anion surfactant, a cation surfactant, and an amphoteric surfactant, a natural water-soluble polymer, a semisynthetic water-soluble polymer, a synthetic water-soluble polymer, an inorganic water-soluble polymer, a preservative, a pH adjuster, an antioxidant, an antioxidizing auxiliary, or a perfume, which will be described later. It is either possible to use a single type, or a combination of two or more types, of these components.

The trehalose fatty acid ester composition of the present invention is able to exert a moderate hardening effect dependently on the blending amount, when added to a wax. This hardening effect dependent on the blending amount can be observed in a wide range from over 0% by mass to about 80% by mass, in terms of the blending ratio of the trehalose fatty acid ester composition of the present invention to the wax, relative to the total amount of the solidifiers. In other words, the ratio of the amount of a wax can be sufficiently lowered by blending the trehalose fatty acid ester composition of the present invention into the wax, without deteriorating the hardness.

As will be described in Comparative Examples later, if a sucrose fatty acid ester usually available in the market is added to a wax; the hardness is decreased lower than the hardness before the addition of the sucrose fatty acid ester when the blending amount of the sucrose fatty acid ester is small; then the hardness is rapidly increased along with the increase of the blending ratio; and the hardness is rapidly decreased when the blending ratio is much increased. In this way, the fluctuation of the hardness with the variation of the blending ratio is so sharp that it is very difficult to determine the appropriate blending ratio of the sucrose fatty acid ester to the wax. On the other hand, in the case of the trehalose fatty acid ester composition of the present invention, the fluctuation of the hardness of the wax composition along with the variation of the blending ratio to the wax is moderate. Moreover, it is also possible to increase the hardness even by adding a small amount to various types of waxes. In other words, the trehalose fatty acid ester composition of the present invention has an excellent effect for adjusting the hardness, and enables to readily determine a suitable blending amount for adjusting the resultant wax composition to have a desired hardness.

Furthermore, the trehalose fatty acid ester composition of the present invention has high thermostability. For this reason, the trehalose fatty acid ester composition of the present invention is able to suppress the denaturation induced by heating at the time of the production, even if the composition is used as a raw material of a cosmetic or such a product.

In this way, the trehalose fatty acid ester composition of the present invention has high thermostability and exerts an excellent hardening effect in either case where the blending ratio to the wax is largely increased or decreased. For this reason, the trehalose fatty acid ester composition of the present invention is suitable as a crystal modifier, particularly as a crystal modifier to be added to a solid cosmetic which is required to be excellent in both the shape retainability and the sense of use.

The crystal modifier including the trehalose fatty acid ester composition of the present invention may also contain another component as long as the effect of the trehalose fatty acid ester composition of the present invention would not be impaired. For example, the crystal modifier may also contain a wax.

The trehalose fatty acid ester composition of another aspect of the present invention is preferably such that over 90% by mass and 100% by mass or less relative to all the fatty acid residues held by all the trehalose fatty acid esters in the trehalose fatty acid ester composition of the present invention are at least one type of residue selected from the group consisting of a palmitic acid residue and a stearic acid residue, over 50% by mass and 100% by mass or less of all the fatty acid residues are a stearic acid residue, and the sum amount of a tetraester, a pentaester, and a hexaester, relative to the total peak area, calculated by HPLC analysis, is from 50 to 70% by area. If the sum amount of a tetraester, a pentaester, and a hexaester, relative to the total peak area, calculated by HPLC analysis, is from 50 to 70% by area, it is preferable to blend the trehalose fatty acid ester composition of the present invention as a solidifier of a solid form cosmetic at 5 to 80% by mass, relative to the total amount of solidifiers.

The trehalose fatty acid ester composition of yet another aspect of the present invention is preferably such that over 90% by mass and 100% by mass or less relative to all the fatty acid residues held by all the trehalose fatty acid esters in the trehalose fatty acid ester composition of the present invention are at least one type of residue selected from the group consisting of a palmitic acid residue and a stearic acid residue, over 50% by mass and 100% by mass or less of all the fatty acid residues are a stearic acid residue, and the sum amount of a hexaester and a heptaester, relative to the total peak area, calculated by HPLC analysis, is 50% by area or more. If the sum amount of a hexaester and a heptaester, relative to the total peak area, calculated by HPLC analysis, is from 50% by area or more, it is preferable to blend the trehalose fatty acid ester composition of the present invention as a solidifier of a solid form cosmetic at 5 to 90% by mass, relative to the total amount of solidifiers.

The trehalose fatty acid ester composition of even yet another aspect of the present invention is preferably such that over 90% by mass and 100% by mass or less relative to all the fatty acid residues held by all the trehalose fatty acid esters in the trehalose fatty acid ester composition of the present invention are a palmitic acid residue, and the sum amount of a hexaester and a heptaester, relative to the total peak area, calculated by HPLC analysis, is 50% by area or more. If the sum amount of a hexaester and a heptaester, relative to the total peak area, calculated by HPLC analysis, is 50% by area or more, it is preferable to blend the trehalose fatty acid ester composition of the present invention as a solidifier of a solid form cosmetic at 5 to 90% by mass, relative to the total amount of solidifiers.

### <Wax composition>

The trehalose fatty acid ester composition of the present invention is an excellent crystal modifier for a wax, and is also a solidifier. For this reason, a wax composition which excels in the hardness and the excipient function can be obtained by blending the trehalose fatty acid ester composition of the present invention and the wax, even though the blending ratio of the wax is low.

The wax composition of the present invention contains the trehalose fatty acid ester composition of the present invention and a wax.

The type of the wax in the wax composition of the present invention is not specifically limited, and waxes for usual use in cosmetics, such as a candelilla wax, a carnauba wax, a paraffin wax, a polyethylene wax, and a ceresin wax, can be used.

The wax composition of the present invention may also contain another component in addition to the trehalose fatty acid ester composition of the present invention and a wax. Such another component can be exemplified by an oil solution, a surfactant, a preservative, a pH adjuster, an antioxidant, an antioxidizing auxiliary, or a perfume, which will be described later. It is either possible to use a single type, or a combination of two or more types, of these components

### <Solid cosmetic>

The trehalose fatty acid ester composition of the present invention is an excellent solidifier and is suitable as an additive of a solid cosmetic. It is possible to produce, by blending the trehalose fatty acid ester composition of the present invention, a solid cosmetic which excels in the thermostability at the time of the production as well as having a satisfactory sense of use and make-up lasting.

The solid cosmetic of the present invention comprises the trehalose fatty acid ester composition of the present invention and a wax.

The solid cosmetic can be exemplified by a rouge, a lip cream, a lip gloss, a stick concealer, an eye-color pencil, or a clay wax. It is preferable that the solid cosmetic including the trehalose fatty acid ester composition of the present invention contains a wax.

The amount of the trehalose fatty acid ester composition of the present invention in the solid cosmetic is not specifically limited, and can be appropriately determined with consideration of the types and the blending ratios of other components, the intended property of the product, or the like. For example, it is preferable to contain the trehalose fatty acid ester composition of the present invention at 0.5 to 30% by mass, more preferably at 0.75 to 25% by mass, and yet more preferably at 1 to 20% by mass, relative to the total mass of the solid cosmetic.

The amounts and the blending ratios of the trehalose fatty acid ester composition of the present invention and the wax in the solid cosmetic are not specifically limited, and can be appropriately determined with consideration of the types and the blending ratios of other components, the intended property of the product, or the like. For example, it is preferable to contain the trehalose fatty acid ester composition of the present invention and the wax at the sum amount of 1 to 30% by mass, more preferably 3 to 30% by mass, and yet more preferably 3 to 25% by mass, relative to the total mass of the solid cosmetic.

The trehalose fatty acid ester composition of the present invention may be directly used as a solidifier or as a crystal modifier in the form of a raw material of a solid cosmetic, or may also be added to a solid cosmetic in the form of a wax composition having been previously blended with a wax component. For example, it is preferable to contain the wax composition of the present invention at 1 to 30% by mass, more preferably 3 to 30% by mass, and yet more preferably 3 to 25% by mass, relative to the total mass of the solid cosmetic.

The solid cosmetic including the trehalose fatty acid ester composition of the present invention can be blended with various types of components for usual use in cosmetics and the like, as required, within a range that would not impair the effects of the present invention. The solid cosmetic can be produced by a conventionally known method.

For example, the solid cosmetic can be appropriately blended with an anion surfactant, a cation surfactant, an amphoteric surfactant, a lipophilic nonionic surfactant, a hydrophilic nonionic surfactant, a silicone based surfactant, a natural based surfactant, liquid fats and oils, solid fats and oils, a wax, a hydrocarbon oil, a higher fatty acid, a higher alcohol, an ester oil, a silicon oil, a powder, a moisturizer, a natural water-soluble polymer, a semisynthetic water-soluble polymer, a synthetic water-soluble polymer, an inorganic water-soluble polymer, a thickener, an ultraviolet absorber, a metal ion sequester, a lower alcohol, a polyalcohol, a monosaccharide, an oligosaccharide, a polysaccharide, an amino acid, an organic amine, a synthetic-resin emulsion, a pH adjuster, a vitamin, an antioxidant, an antioxidizing auxiliary, a perfume, water, or the like, as required.

The anion surfactant can be exemplified by: fatty-acid soaps such as substrates for soap, sodium laurate, or sodium palmitate; salts of higher alkyl sulfuric ester such as sodium lauryl sulfate or potassium lauryl sulfate; salts of alkyl ether sulfuric ester such as POE-triethanolamine lauryl sulfate or POE-sodium lauryl sulfate; N-acylsarcosine acids such as sodium lauroyl sarcosine; higher fatty acid amide sulfonates such as sodium N-myristoyl-N-methyl taurate, sodium palm oil fatty acid methyl ester tauride, or sodium lauryl methyl tauride; salts of phosphoric ester such as sodium POE-oleyl ether phosphate or POE-stearyl ether phosphoric acid; sulfosuccinates such as sodium di-2-ethylhexyl sulfosuccinate, sodium monolauroyl monoethanolamide polyoxyethylene sulfosuccinate, or sodium lauryl polypropylene glycol sulfosuccinate; alkylbenzene sulfonates such as linear sodium dodecylbenzenesulfonate, linear triethanolamine dodecylbenzenesulfonate, or linear dodecylbenzenesulfonic acid; N-acylglutamates such as monosodium N-lauroyl glutamate, disodium N-stearoyl glutamate, or monosodium N-myristoyl-L-glutamate; higher fatty acid ester sulfates such as hardened palm oil fatty acid glycerin sodium sulfate; sulfated oils such as sulfated castor oil; POE-alkylether carboxylic acids; POE-alkylallyl ether carboxylates; α-olefin sulfonates; higher fatty acid ester sulfonates; secondary alcohol sulfates; higher fatty acid alkylol amide sulfates; sodium lauroyl monoethanolamide succinates; ditriethanolamine N-palmitoyl aspartate; and casein sodium.

The cation surfactant can be exemplified by alkyl trimethyl ammonium salts such as stearyl trimethyl ammonium chloride or lauryl trimethyl ammonium chloride; alkylpyridinium salts such as distearyl dimethyl ammonium chloride dialkyl dimethyl ammonium salts, poly(N,N'-dimethyl-3,5-methylene piperidinium) chloride, or cetylpyridinium chloride; alkyl quaternary ammonium salts; alkyl dimethyl benzyl ammonium salts; alkyl isoquinolinium salts; dialkyl morphonium salts; POE-alkylamine; alkylamine salts; polyamine fatty acid derivatives; amyl alcohol fatty acid derivatives; benzalkonium chloride; and benzetonium chloride.

The amphoteric surfactant can be exemplified by imidazoline based ampholytic surfactants such as sodium 2-undecyl-N,N,N-(hydroxyethyl carboxymethyl)-2-imidazoline and salts of disodium 2-cocoyl-2-imidazolinium hydroxide-1-carboxyethyloxy; and betaine based ampholytic surfactants such as 2-heptadecyl-N-carboxymethyl-N-hydroxyethyl imidazolinium betaine, lauryl dimethylamino acetic acid betaine, alkyl betaine, amido betaine, and sulfobetaine.

The lipophilic nonionic surfactant can be exemplified by sorbitan fatty acid esters such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, or tetra-2-ethylhexyl diglycerol sorbitan; glycerin fatty acids such as mono cottonseed oil fatty acid glycerin, monoerucic acid glycerin, sesquioleic acid glycerin, monostearic acid glycerin, α,α'-oleic acid pyroglutamic acid glycerin, or monostearic acid glycerin; polyglycerin fatty acid esters such as diglyceryl monoisostearate or diglyceryl diisostearate; propylene glycol fatty acid esters such as propylene glycol monostearate; hardened castor oil derivatives; and glycerin alkylethers.

The hydrophilic nonionic surfactant can be exemplified by POE-sorbitan fatty acid esters such as POE-sorbitan monooleate, POE-sorbitan monostearate, POE-sorbitan monooleate, or POE-sorbitan tetraoleate; POE-sorbit fatty acid esters such as POE-sorbit monolaurate, POE-sorbit monooleate, POE-sorbit pentaoleate, or POE-sorbit monostearate; POE-glycerin fatty acid esters such as POE-glycerin monostearate, POE-glycerin monoisostearate, or POE-glycerin triisostearate; POE-fatty acid esters such as POE-monooleate, POE-distearate, POE-monodioleate, or distearic acid ethylene glycol; POE-alkylethers such as POE-laurylether, POE-oleylether, POE-stearylether, POE-behenylether, POE-2-octyldodecylether, or POE-cholestanolether; pluronic types such as pluronic; POE-POP-alkylethers such as POE-POP-cetylether, POE-POP-2-decyltetradecylether, POE-POP-monobutylether, POE-POP-hydrogenated lanolin, or POE-POP-glycerinether; tetra POE-tetra POP-ethylenediamine condensation products such as tetronic; POE-castor oil hardened castor oil derivatives such as POE-castor oil, POE-hardened castor oil, POE-hardened castor oil monoisostearate, POE-hardened castor oil triisostearate, POE-hardened castor oil monopyroglutamic acid monoisostearic acid diester, or POE-hardened castor oil maleic acid; POE-beeswax-lanolin derivatives such as POE-sorbit beeswax; alkanolamides such as palm oil fatty acid diethanolamide, monoethanolamide laurate, or fatty acid isopropanolamide; POE-propylene glycol fatty acid esters; POE-alkylamines; POE-fatty acid amides; sucrose fatty acid esters; POE-nonylphenyl formaldehyde condensation products; alkylethoxy dimethyl amine oxides; and trioleyl phosphoric acids.

The silicone based surfactant can be exemplified by polyether-modified polysiloxane, a polyoxyalkylene alkylmethyl polysiloxane-methyl polysiloxane copolymer, or alkoxy modified polysiloxane.

The natural based surfactant can be exemplified by lecithins such as soybean phospholipids, hydrogenated soybean phospholipids, egg yolk phospholipids, or hydrogenated egg yolk phospholipids; and soybean saponins.

The liquid fats and oils can be exemplified by avocado oil, camellia oil, turtle oil, macadamia nut oil, corn oil, sunflower oil, mink oil, olive oil, canola oil, egg yolk oil, sesame seed oil, persic oil, wheat germ oil, sasanqua oil, castor oil, linseed oil, safflower oil, grapeseed oil, cottonseed oil, perilla oil, soybean oil, earthnut oil, tea seed oil, torreya seed oil, rice bran oil, aleurites fordii oil, Japanese tung oil, jojoba oil, germ oil, evening primrose oil, trioctanoic acid glycerin, and triisopalmitic acid glycerin. Here, the liquid fats and oils mean fats and oils which are liquid at room temperature.

The solid fats and oils can be exemplified by cacao butter, palm oil, beef tallow, mutton tallow, horse fat, palm kernel oil, lard, beef bone fat, tree wax kernel oil, hoof oil, tree wax, hardened coconut oil, hardened palm oil, hardened beef tallow, hardened oil, and hardened castor oil.

The wax can be exemplified by beeswax, candelilla wax, cotton wax, carnauba wax, bayberry wax, *Ericerus pela* wax, whale wax, montan wax, rice bran wax, kapok wax, sugarcane wax, lanolin, acetylated lanolin, liquid lanolin, isopropyl lanolate, reduced lanolin, hard lanolin, hexyl laurate, jojoba wax, shellac wax, POE lanolin alcohol ether, POE lanolin alcohol acetate, POE cholesterol ether, lanolin fatty acid polyethylene glycol, and POE hydrogenated lanolin alcohol ether.

The hydrocarbon oil can be exemplified by liquid paraffin, isoparaffin, heavy liquid isoparaffin, paraffin, ozocerite, squalane, vegetable squalane, pristine, ceresin, squalene, vaseline, microcrystalline wax, paraffin wax, montan wax, olefin oligomer, polyisobutylene, polybutene, and hydrogenated polybutene.

The higher fatty acid can be exemplified by lauric acid, myristic acid, palmitic acid, stearic acid, behenic acid, oleic acid, undecylenic acid, tall oil acid, isostearic acid, linoleic acid, linolenic acid, eicosapentaenoic acid (EPA), and docosahexaenoic acid (DHA).

The higher alcohol can be exemplified by linear alcohols such as lauryl alcohol, cetyl alcohol, stearyl alcohol, behenyl alcohol, myristyl alcohol, oleyl alcohol, and setostearyl alcohol; and branched alcohols such as monostearyl glycerin ether (batyl alcohol), 2-decyl tetradecynol, lanolin alcohol, cholesterol, phytosterol, hexyl dodecanol, isostearic alcohol, and octyl dodecanol.

The ester oil can be exemplified by isopropyl myristate, cetyl isooctanoate, octyldodecyl myristate, isopropyl palmitate, isooctyl palmitate, butyl stearate, hexyl laurate, myristyl myristate, decyl oleate, hexyldecyl dimethyl octanoate, cetyl lactate, myristyl lactate, octyldodecyl lactate, acetylated lanolin, isocetyl stearate, isocetyl isostearate, cholesteryl 12-hydroxy stearate, phytosteryl 12-hydroxy stearate, phytosteryl oleate, ethylene glycol di-2-ethylhexanoate, propylene glycol dicaprate, dipentaerythritol fatty acid ester, N-alkyl glycol mono isostearate, neopentyl glycol dicaprate, diisostearyl malate, glycerin di-2-heptyl undecanoate, trimethylolpropane tri-2-ethylhexanoate, trimethylolpropane triisostearate, dipentaerythrityl tri-polyhydroxystearate, pentaerythritol tetraisostearate, dipentaerythrityl tetraisostearate, dipentaerythrityl pentaisostearate, pentaerythritol tetra-2-ethylhexanoate, glyceryl tri-2-ethylhexanoate, tri(caprylic/capric acid) glyceryl, tri(caprylic/capric/myristic/stearic acid) glyceride, propanediol dicaprylate/dicaprate, propanediol diisostearate, trimethylolpropane triisostearate, cetyl 2-ethylhexanoate, 2-ethylhexylpalmitate, glycerin trimyristate, tri-2-heptyl undecanoic acid glyceride, polyglyceryl diisostearate, polyglyceryl triisostearate, polyglyceryl tetraisostearate, diglyceryl triisostearate, diglyceryl tetraisostearate, erythrityl tri-2-ethylhexanoate, ditrimethylolpropane tri-2-ethylhexanoate, (isostearic acid/sebacic acid) ditrimethylolpropane oligoester, castor oil fatty acid methyl ester, oleyl oleate, acetoglyceride, 2-heptyl undecyl palmitate, diisobutyl adipate, (adipic acid/2-ethylhexanoic acid/stearic acid) glycerin oligoester, (2-hexyl decanoic acid/sebacic acid) diglyceryl oligoester, N-lauroyl-L-glutamic acid-2-octyldodecylester, di-2-heptyl undecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, and triethyl citrate.

The silicon oil can be exemplified by: chain polysiloxanes such as dimethyl polysiloxane, methylphenyl polysiloxane, or methylhydrogen polysiloxane; cyclic polysiloxanes such as octamethylcyclotetrasiloxane, decamethylcyclopentasiloxane, dodecamethyl-cyclohexasiloxane, or tetrahydrotetramethylcyclotetrasiloxane; and polyoxyethylene polyalkyl siloxane.

The usability of the cosmetics containing the trehalose fatty acid ester composition of the present invention can be improved and the toning of the cosmetics can be adjusted by adding a powder. Moreover, the powder that can be used herein is not particularly limited by the shape such as spherical, plate-like, and needle-like shapes, the particle size such as the sizes of fumy particles, fine particles, and pigment particles, and the particle structure such as porous and non-porous structures, and it is possible to use inorganic powders, photoluminescent powders, organic powders, dye powders, metal powders, and composite powders.

Specific examples of the powder include white inorganic pigments such as titanium oxide, zinc oxide, cerium oxide, or barium sulfate; colored inorganic pigment powders such as ferric oxide, titanic iron, γ-ferric oxide, iron oxide yellow, iron oxide black, carbon black, low-dimensional titanic oxide, chrome oxide, chromium hydroxide, iron blue, ultramarine blue, yellow ocher, manganese violet, cobalt violet, or titanic cobalt; organic pigment powders such as Red No. 201, Red No. 202, Red No. 205, Red No. 220, Red No. 226, Red No. 228, Red No. 405, Orange No. 203, Orange No. 204, Blue No. 404, Yellow No. 205, or Yellow No. 401; and colored organic pigment powders such as zirconium, barium, or aluminum lake, e.g., Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, or Blue No. 1. Examples of the extenders include: white extender powders such as talc, mica, white mica, gold mica, red mica, black mica, synthesized mica, sericite, lithia mica, vermiculite, synthesized sericite, kaolin, silicon carbide, bentonite, smectite, aluminum oxide, magnesium oxide, zirconium oxide, antimony oxide, diatom earth, aluminum silicate, magnesium aluminum metasilicate, calcium silicate, barium silicate, magnesium silicate, strontium silicate, metal salts of tungsten acid, calcium phosphate, calcium carbonate, magnesium carbonate, calcined calcium sulfate, apatite fluoride, hydroxyapatite, silica, zeolite, ceramic powder, or boron nitride; photoluminescent powders such as titanium dioxide coated mica, titanium dioxide coated mica, titanium dioxide coated talc, titanium dioxide coated bismuth oxychloride, colored titanium oxide coated mica, ferric oxide mica titanium, iron blue treated mica titanium, carmine treated mica titanium, bismuth oxychloride, argentine, polyethylene telephthalate/aluminum/epoxy laminated powder, or polyethylene telephthalate/polyolefin laminated powder; copolymer resins such as polyamide based resins, polyethylene based resins, polyacryl based resins, polyester based resins, fluorine based resins, cellulose based resins, polystyrene based resins, or styrene-acryl copolymer resins; organic polymer resin powders such as polypropylene based resins, silicone resins, urethane resins, benzoguanamine resins, or polyethylene tetrafluoride resins; organic low molecular powders such as zinc myristate, zinc stearate, calcium palmitate, aluminum stearate, or N-acyllysine; natural organic powders such as starch, silk powder, or cellulose powder; or, additionally, metal powders such as aluminum powder, magnesium powder, copper powder, gold powder, or silver powder; or compound powders such as particulate titanium oxide coated mica titanium, particulate zinc oxide coated mica titanium, barium sulfate coated mica titanium, silicon dioxide containing titanium oxide, or silicon dioxide containing zinc oxide. These powders can be used alone, or in a combination of two or more types thereof, and a complex compound thereof can also be used. These powders can be used after surface treatment with one or more types of substances selected from fluorine based compounds, silicone based compounds, metal soaps, lecithins, hydrogenated lecithins, collagen, hydrocarbons, higher fatty acids, higher alcohols, esters, waxes, and surfactants.

The moisturizer can be exemplified by polyethylene glycol, propylene glycol, 1,3-propanediol, glycerin, 1,3-butylene glycol, xylitol, sorbitol, maltitol, chondroitin sulfuric acid, hyaluronic acid, mucoitinsulfuric acid, charonic acid, atelocollagen, cholesteryl-12-hydroxy stearate, sodium lactate, urea, bile salt, dl-pyrrolidone carboxylate, short-chain soluble collagen, diglycerin (EO) PO adducts, extracts of rosa roxburghii, yarrow extracts, and melilot extracts.

The natural water-soluble polymer can be exemplified by: plant-based polymers such as gum arabic, gum tragacanth, galactan, guar gum, gum carob, Karaya gum, carrageenan, pectin, agar, quince seed (marmelo), algae colloid (brown algae extracts), or starch (rice, corn, potato, or wheat); microbial-based polymers such as xanthan gum, dextran, succinoglucan, or pullulan; or animal-based polymers such as collagen, casein, albumin, or gelatin.

The semisynthetic water-soluble polymer can be exemplified by: starch based polymers such as carboxymethyl starch or methylhydroxypropyl starch; cellulose based polymers such as methyl cellulose, nitrocellulose, methylhydroxypropyl cellulose, cellulose sodium sulfate, hydroxypropyl cellulose, carboxymethyl cellulose, sodium carboxymethyl cellulose, crystalline cellulose, or cellulose powder; and alginic acid based polymers such as sodium alginate or alginic acid propylene glycol ester.

The synthetic water-soluble polymer can be exemplified by: vinyl based polymers such as polyvinyl alcohol, polyvinyl methyl ether, polyvinyl pyrrolidone, or carboxyvinyl polymer (carbopol); polyoxyethylene based polymers such as polyethylene glycol 20,000, 40,000, or 60,000; acrylic based polymers such as polyoxyethylene polyoxypropylene copolymer copolymerized polymer, polyacrylic acid sodium, polyethyl acrylate, or polyacrylamide; polyethylene imine; and cation polymer.

The inorganic water-soluble polymer can be exemplified by bentonite, AlMg silicate (bee gum), laponite, hectorite, and anhydrous silicic acid.

The thickener can be exemplified by gum arabic, carrageenan, Karaya gum, gum tragacanth, carob gum, quince seed (marmelo), casein, dextrin, gelatin, sodium pectin acid, sodium alginate, methylcellulose, ethylcellulose, CMC, hydroxyethyl cellulose, hydroxypropyl cellulose, PVA, PVM, PVP, sodium polyacrylate, carboxyvinyl polymer, locust bean gum, guar gum, tamarind gum, dialkyl dimethyl ammonium cellulose sulfate, xanthan gum, magnesium aluminum silicate, bentonite, and hectorite.

The ultraviolet absorber can be exemplified by: benzoic acid based ultraviolet absorbers such as para-aminobenzoic acid (hereinafter abbreviated as PABA), PABA mono glycerin ester, N,N-dipropoxy PABA ethyl ester, N,N-diethoxy PABA ethyl ester, N,N-dimethyl PABA ethyl ester, N,N-dimethyl PABA butyl ester, or N,N-dimethyl PABA ethyl ester; anthranilic acid based ultraviolet absorbers such as homomethyl-N-acetylanthranilate; salicylic acid based ultraviolet absorbers such as amyl salicylate, menthyl salicylate, homomethyl salicylate, octyl salicylate, phenyl salicylate, benzyl salicylate, or p-isopropanol phenyl salicylate; cinnamic acid based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, or glyceryl mono-2-ethylhexanoyl-diparamethoxycinnamate; benzophenone based ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, or 4-hydroxy-3-carboxybenzophenone; 3-(4'-methylbenzylidene)d,l-camphor; 3-benzyhdene-d,l-camphor; urocanic acid; urocanic acid ethyl ester; 2-phenyl-5-methylbenzoxazole; 2,2'-hydroxy-5-methylphenyl benzotriazole; 2-(2'-hydroxy-5'-t-octylphenyl) benzotriazole; 2-(2'-hydroxy-5'-methylphenyl) benzotriazole; dibenzalazine; dianisoylmethane; 4-methoxy-4'-t-butyl dibenzoylmethane; 5-(3,3-dimethyl-2-norbornylidene)-3-pentane-2-one; and 2,4,6-trianilino-p-(carbo-2'-ethylhexyl-1'-oxy) 1,3,5-triazine.

The metal ion sequestrant can be exemplified by 1-hydroxyethane-1,1-diphosphonate, tetrasodium salt of 1-hydroxyethane-1,1-diphosphonate, disodium edentate, edetate trisodium, edentate tetrasodium, sodium citrate, sodium polyphosphate, sodium metaphosphate, gluconic acid, phosphoric acid, citric acid, ascorbic acid, succinic acid, edetic acid, and trisodium ethylenediamine hydroxyethyl triacetate.

The lower alcohol can be exemplified by methanol, ethanol, propanol, isopropanol, isobutyl alcohol, and t-butyl alcohol.

The polyalcohol can be exemplified by: dihydric alcohols such as ethylene glycol, propylene glycol, trimethylene glycol, 1,2-butylene glycol, 1,3-butylene glycol, tetramethylene glucol, 2,3-butylene glucol, pentamethylene glucol, 2-butene-1,4-diol, hexylene glycol, 1,3-propanediol, or octylene glycol; trihydric alcohols such as glycerin, trimethylolpropane, or 1,2,6-hexanetriol; tetrahydric alcohols such as pentaerythritol or erythritol; pentahydric alcohols such as xylitol; hexahydric alcohols such as sorbitol, mannitol, or inositol; polyalcohol polymers such as diethylene glycol, dipropylene glycol, triethylene glucol, polypropylene glycol, tetraethylene glycol, diglycerin, polyethylene glycol, triglycerin, tetraglycerin, or polyglycerin; dihydric alcoholic alkyl ethers such as ethylene glycol monomethyl ether, ethylene glucol monoethyl ether, ethylene glycol monobutyl ether, ethylene glycol monophenyl ether, ethylene glycol monohexyl ether, ethylene glycol mono 2-methylhexyl ether, ethylene glycol isoamyl ether, ethylene glycol benzyl ether, ethylene glycol isopropyl ether, ethylene glycol dimethyl ether, ethylene glycol diethyl ether, or ethylene glycol dibutyl ether; dihydric alcohol alkyl ethers such as diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, or dipropylene glycol butyl ether; dihydric alcohol ether esters such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, or propylene glycol monophenyl ether acetate; glycerin monoalkyl ethers such as xyl alcohol, selachyl alcohol, or batyl alcohol; sugar alcohols such as sorbitol, maltitol, maltotriose, mannitol, inositol, lactitol, sucrose, raffinose, xylose, glucose, fructose, amylolytic sugar, maltose, xylitose, erythritol, or amylolytic sugar reducing alcohol; glysolid; tetrahydroflufuryl alcohol; POE-tetrahydroflufuryl alcohol; POP-butyl ether; POP/POE-butyl ether; tripolyoxy propylene glycerin ether; POP-glycerin ether; POP-glycerin ether phosphoric acid; and POP/POE-pentane erythritol ether.

The monosaccharide can be exemplified by: trioses such as D-glyceryl aldehyde or dihydroxy acetone; tetroses such as D-erythrose, D-erythrulose, or D-threose; pentoses such as L-arabinose, D-xylose, L-lyxose, D-arabinose, D-ribose, D-ribulose, D-xylulose, or L-xylulose; hexoses such as D-glucose, D-talose, D-psicose, D-galactose, D-fructose, L-galactose, L-mannose, or D-tagatose; heptoses such as aldoheptose or heptrose; octoses such as octrose; deoxy sugars such as 2-deoxy-D-ribose, 6-deoxy-L-galactos, or 6-deoxy-L-mannose; amino sugars such as D-glucosamine, D-galactosamine, sialic acid, amino uronic acid, or muramic acid; and uronic acids such as D-glucuronic acid, D-mannuronic acid, L-guluronic acid, D-galacturonic acid, or L-iduronic acid.

The oligosaccharide can be exemplified by sucrose, gunchianose, umbelliferose, lactose, planteose, isolignoses, α,α-trehalose, raffinose, lignoses, umbilicine, and stachyose verbascoses.

The polysaccharide can be exemplified by cellulose, quince seed, chondroitin sulfuric acid, starch, dextrin, glucomannan, chitin, galactan, dermatan sulfuric acid, glycogen, gum arabic, heparin sulfuric acid, hyaluronic acid, gum tragacanth, keratan sulfuric acid, chondroitin, xanthan gum, mucoitinsulfuric acid, guar gum, dextran, keratosulfuric acid, locust bean gum, succinoglucan, and caronic acid.

The amino acid can be exemplified by: neutral amino acids such as threonine or cysteine; or basic amino acids such as hydroxylysine. Moreover, amino acid derivatives can be exemplified by sodium acylsarcosine (sodium lauroylsarcosine), acyl glutamate, acyl β-alanine sodium, glutathione, and pyrrolidone carboxylic acid.

The organic amine can be exemplified by monoethanolamine, diethanolamine, triethanolamine, morpholine, triisopropanolamine, 2-amino-2-methyl-1,3-propanediol, and 2-amino-2-methyl-1-propanol.

The synthetic resin emulsion can be preferably exemplified by an acrylic resin emulsion, a polyacrylic acid ethyl emulsion, an acrylic resin solution, a polyacryl alkyl ester emulsion, and a polyvinyl acetate resin emulsion.

The pH adjuster can be exemplified by buffers such as lactic acid-sodium lactate, and citric acid-sodium citrate.

The vitamins can be exemplified by vitamin A, vitamin B1, vitamin B2, vitamin B6, vitamin C and derivatives thereof, vitamin E, vitamin K and derivatives thereof, pantothenic acids and derivatives thereof, biotins, and the like.

The antioxidant can be exemplified by tocopherols, dibutyl hydroxytoluene, butylhydroxyanisol, and gallic acid esters.

The antioxidizing auxiliary can be exemplified by phosphoric acids, citric acids, ascorbic acids, maleic acids, malonic acids, succinic acids, fumaric acids, cephalin, hexametaphosphate, phytic acid, and ethylenediamine tetraacetic acid.

The other blendable components can be exemplified by: antiseptic agents such as ethylparaben or butylparaben; ultraviolet absorbers such as benzophenone derivatives, PABA derivatives, cinnamic acid derivatives, salicylic acid derivatives, 4-tert-butyl-4'-methoxydibenzoylmethane, or oxybenzone; anti-inflammatory agents such as glycyrrhizinic acid derivatives, glycyrrhetinic acid derivatives, salicylic acid derivatives, hinokitiol, zinc oxide, or allantoin; skin whitening agents such as placental extracts, vitamin C and derivatives thereof, hydroquinone and derivatives thereof, and saxifragaceous extracts; extracts of cork tree bark, coptis root, lithospermi radix, peony root, swertia herb, birch, sage, loquat, carrots, aloe, tree mallow, iris, grapes, coix seed, loofah, lily, saffron, Cnidium Rhizome, ginger, hypericum, ononis, garlic, capsicum, citrus unshiu peel, Japanese angelica root, or seaweed; activator agents such as royal jelly, photosensitive pigments, cholesterol derivatives, and infant blood extracts; blood circulation promoters such as nonylic acid vanillyl amide, nicotinic acid benzyl ester, nicotinic acid β-butoxy ethyl ester, capsaicin, gingerone, cantharides tincture, ichthammol, tannic acid, α-borneol, nicotinic acid tocopherol, inositol hexanicotinate, cyclandelate, cinnarizine, tolazoline, acetylcholine, verapamil, cepharanthine, or γ-orizanol; antiseborrheic agents such as sulfur or thianthol; tranexamic acids; thiotaurine; and hypotaurine.

### [Examples]

Hereunder is a more detailed description of the present invention with reference to specific Examples. Note that, the present invention is in no way limited by the contents of the following Examples.

### <Methods for Analyzing and Measuring Trehalose Fatty Acid Ester Composition and Sucrose Fatty Acid Ester Composition>

### [Measurement Conditions of High Performance Liquid Chromatography (HPLC) Analysis]

### (Measurement condition A)

The measurement condition of HPLC analysis to calculate the % by area of a monoester, a diester, a triester, a tetraester, and a polyester in the trehalose fatty acid ester composition is such that;
Column: Four styrene divinylbenzene-based GPC columns, connected in a series, each being 7.8 mm in inner diameter; 300 mm in length, and 5 µm in size
Mobile phase: Tetrahydrofuran
Column temperature: 40°C
Flow rate of mobile phase: 0.5 mL/min
Detection: Differential refraction index (RI)

### (Measurement condition B)

The measurement condition of HPLC analysis to calculate the ratio of a pentaester, a hexaester, a heptaester, and an octaester in the polyester in the trehalose fatty acid ester composition is such that;
Column: Two ODS columns, connected in a series, each being 4.6 mm in inner diameter; 150 mm in length, and 5 µm in size
Mobile phase: Tetrahydrofuran:Methanol = 50:50 (volume ratio)
Column temperature: 40°C
Flow rate of mobile phase: 1.0 mL/min
Detection: Differential refraction index (RI)

### <Method for calculating Area Percentage (% by Area) of Each Ester>

### (1) Method for calculating the % by area of a monoester, a diester, a trimester, and a tetraester

The percentage of each peak area of raw materials, a monoester, a diester, a triester, and a tetraester, relative to the total peak area, obtained by the measurement by means of HPLC analysis using the GPC columns under the measurement condition A, is taken as the % by area of each ester.

### (2) Method for calculating the % by area of a polyester

The percentage (X) of the sum peak area of the components other than the raw materials, the monoester, the diester, the triester, and the tetraester, relative to the total peak area, obtained by the measurement by means of HPLC analysis using the GPC columns under the measurement condition A, is taken as the % by area of a polyester.

### (3) Method for calculating the ratio of a pentaester, a hexaester, a heptaester, and an octaester in a polyester

The sum peak area of a pentaester, a hexaester, a heptaester, and an octaester, obtained by the measurement by means of HPLC analysis using the ODS column under the measurement condition B, is taken as (Y), and the ratio of each peak area of the pentaester, the hexaester, the heptaester, and the octaester, relative to (Y) is respectively calculated and taken as the ratio of the pentaester, the hexaester, the heptaester, and the octaester in the polyester.

### (4) Method for calculating the % by area of a pentaester, a hexaester, a heptaester, and an octaester

The value obtained by respectively multiplying the % by area (X) of the polyester as determined in (2) with the ratio of each peak area of the pentaester, the hexaester, the heptaester, and the octaester in the polyester as calculated in (3), is taken as the % by area of the pentaester, the hexaester, the heptaester, and the octaester.

### (Synthesis Example 1)

### Trehalose Fatty Acid Ester Composition 1 Obtained by Transesterifying Trehalose with Methyl Stearate

117.7 g (1 mole) of trehalose dihydrate (manufactured by Hayashibara Group., Treha powder), 882.3 g (9.5 moles) of methyl stearate (manufactured by Wako Pure Chemical Industries, Ltd., methyl stearate), and 29.4 g of fatty acid sodium (manufactured by Miyoshi Oil & Fat Co., Ltd., TP-NA) were charged in a 2000 mL four-neck flask equipped with a stirrer, a thermometer, a cork-stoppered nitrogen gas inlet tube, and a cork-stoppered glass tube. While the mixture was stirred at 100°C, the moisture was dried off under reduced pressure. The inner pressure of the four-neck flask was returned back to the normal pressure by using a nitrogen gas. Then, 10.59 g of potassium carbonate serving as a catalyst (manufactured by Wako Pure Chemical Industries, Ltd., potassium carbonate) was added thereto, and the pressure was reduced again. The mixture was allowed to react by stirring at 110°C to 160°C for 60 hours under reduced pressure. After the completion of the reaction, the mixture was diluted with 200 g of xylene. The diluted matter was neutralized by adding warm water and 57.3 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 536 g of the target trehalose fatty acid ester composition 1 having a hydroxyl value of 35 and a saponification value of 174 was obtained.

### (Synthesis Example 2)

### Trehalose Fatty Acid Ester Composition 2 Obtained by Transesterifying Trehalose with Methyl Stearate

167.5 g (1 mole) of trehalose dihydrate (manufactured by Hayashibara Group., Treha powder), 832.5 g (6.3 moles) of methyl stearate (manufactured by Wako Pure Chemical Industries, Ltd., methyl stearate), and 33.5 g of fatty acid sodium (manufactured by Miyoshi Oil & Fat Co., Ltd., TP-NA) were charged in a 2000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 10.05 g of potassium carbonate serving as a catalyst (manufactured by Wako Pure Chemical Industries, Ltd., potassium carbonate) was added thereto, and the pressure was reduced again. The mixture was allowed to react by stirring at 110°C to 145°C for 33 hours under reduced pressure. After the completion of the reaction, the mixture was diluted with 200 g of xylene. The diluted matter was neutralized by adding warm water and 54.7 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 622 g of the target trehalose fatty acid ester composition 2 having a hydroxyl value of 94 and a saponification value of 167 was obtained.

### (Synthesis Example 3)

### Trehalose Fatty Acid Ester Composition 3 Obtained by Transesterifying Trehalose with Methyl Palmitate and Methyl Stearate

275.9 g (1 mole) of trehalose dihydrate (manufactured by Hayashibara Group., Treha powder), 537.5 g (2.73 moles) of methyl palmitate (manufactured by Emery Oleochemicals, EDENOR ME C16-98), 1386.6 g (6.37 moles) of methyl stearate (manufactured by Wako Pure Chemical Industries, Ltd., methyl stearate), and 69.5 g of fatty acid sodium (manufactured by Miyoshi Oil & Fat Co., Ltd., TP-NA) were charged in a 3000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 16.55 g of potassium carbonate serving as a catalyst (manufactured by Wako Pure Chemical Industries, Ltd., potassium carbonate) was added thereto, and the pressure was reduced again. The mixture was allowed to react by stirring at 110°C to 180°C for 50 hours under reduced pressure. After the completion of the reaction, the mixture was diluted with 330 g of xylene. The diluted matter was neutralized by adding warm water and 107.5 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 1320 g of the target trehalose fatty acid ester composition 3 having a hydroxyl value of 28 and a saponification value of 180 was obtained.

### (Synthesis Example 4)

### Trehalose Fatty Acid Ester Composition 4 Obtained by Transesterifying Trehalose with Methyl Palmitate and Methyl Stearate

379.9 g (1 mole) of trehalose dihydrate (manufactured by Hayashibara Group., Treha powder), 508.4 g (1.875 moles) of methyl palmitate (manufactured by Emery Oleochemicals, EDENOR ME C16-98), 1311.6 g (4.375 moles) of methyl stearate (manufactured by Wako Pure Chemical Industries, Ltd., methyl stearate), and 95.0 g of fatty acid sodium (manufactured by Miyoshi Oil & Fat Co., Ltd., TP-NA) were charged in a 3000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 22.8 g of potassium carbonate serving as a catalyst (manufactured by Wako Pure Chemical Industries, Ltd., potassium carbonate) was added thereto, and the pressure was reduced again. The mixture was allowed to react by stirring at 110°C to 145°C for 30 hours under reduced pressure. After the completion of the reaction, the mixture was diluted with 330 g of xylene. The diluted matter was neutralized by adding warm water and 148.1 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 1420 g of the target trehalose fatty acid ester composition 4 having a hydroxyl value of 99 and a saponification value of 169 was obtained.

### (Synthesis Example 5)

### Trehalose Fatty Acid Ester Composition 5 Obtained by Transesterifying Trehalose with Methyl Palmitate

285.9 g (1 mole) of trehalose dihydrate (manufactured by Hayashibara Group., Treha powder), 1714.1 g (8.4 moles) of methyl palmitate (manufactured by Emery Oleochemicals, EDENOR ME C16-98), and 71.5 g of fatty acid sodium (manufactured by Miyoshi Oil & Fat Co., Ltd., TP-NA) were charged in a 3000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 17.15 g of potassium carbonate serving as a catalyst (manufactured by Wako Pure Chemical Industries, Ltd., potassium carbonate) was added thereto, and the pressure was reduced again. The mixture was allowed to react by stirring at 110°C to 190°C for 72 hours under reduced pressure. After the completion of the reaction, the mixture was diluted with 300 g of xylene. The diluted matter was neutralized by adding warm water and 111.3 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 1288 g of the target trehalose fatty acid ester composition 5 having a hydroxyl value of 22 and a saponification value of 195 was obtained.

### (Synthesis Example 6)

### Trehalose Fatty Acid Ester Composition 6 Obtained by Transesterifying Trehalose with Methyl Palmitate

254.7 g (1 mole) of trehalose dihydrate (manufactured by Hayashibara Group., Treha powder), 1145.3 g (6.3 moles) of methyl palmitate (manufactured by Emery Oleochemicals, EDENOR ME C16-98), and 50.9 g of fatty acid sodium (manufactured by Miyoshi Oil & Fat Co., Ltd., TP-NA) were charged in a 2000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 15.28 g of potassium carbonate serving as a catalyst (manufactured by Wako Pure Chemical Industries, Ltd., potassium carbonate) was added thereto, and the pressure was reduced again. The mixture was allowed to react by stirring at 110°C to 150°C for 36 hours under reduced pressure. After the completion of the reaction, the mixture was diluted with 210 g of xylene. The diluted matter was neutralized by adding warm water and 89.2 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 964 g of the target trehalose fatty acid ester composition 6 having a hydroxyl value of 89 and a saponification value of 184 was obtained.

### (Synthesis Example 7)

### Trehalose Fatty Acid Ester Composition 7 Obtained by Transesterifying Trehalose with Methyl Palmitate

231.2 g (1 mole) of trehalose dihydrate (manufactured by Hayashibara Group., Treha powder), 486.8 g (3.2 moles) of methyl palmitate (manufactured by Emery Oleochemicals, EDENOR ME C16-98), and 500 g of dimethyl sulfoxide (manufactured by Wako Pure Chemical Industries, Ltd., dimethyl sulfoxide) were charged in a 2000 mL four-neck flask equipped with a stirrer, a thermometer, a cork-stoppered nitrogen gas inlet tube, and a cork-stoppered glass tube. The mixture was stirred at 70°C while supplying a nitrogen gas so as to dissolve the trehalose. Thereafter, the mixture was stirred at 70°C to 80°C for 1 hour to be dried under reduced pressure. The inner pressure of the four-neck flask was returned back to the normal pressure by using a nitrogen gas. Then, 6.39 g of potassium carbonate serving as a catalyst (manufactured by Wako Pure Chemical Industries, Ltd., potassium carbonate) was added thereto, and the pressure was reduced again. The mixture was allowed to react by stirring at 70°C to 130°C for 30 hours under reduced pressure. After the completion of the reaction, the mixture was diluted with 300 g of xylene. The diluted matter was neutralized by adding warm water and 20.7 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 490 g of the target trehalose fatty acid ester composition 7 having a hydroxyl value of 242 and a saponification value of 160 was obtained.

### (Synthesis Example 8)

### Trehalose Fatty Acid Ester Composition 8 Obtained by Transesterifying Trehalose with Methyl Stearate and Isomethyl Stearate

207.9 g (0.55 moles) of trehalose dihydrate (manufactured by Hayashibara Group., Treha powder), 493.1 g (1.65 moles) of methyl stearate (manufactured by Wako Pure Chemical Industries, Ltd., methyl stearate), 496.7 g (1.67 moles) of isomethyl stearate (having an acid value of 2.0, prepared by a usual method), 71.9 g of isostearic acid (manufactured by Cognis Corporation, Emersol 874), and 111.3 g of 10 wt % aqueous solution of sodium hydroxide were charged in a 2000 mL four-neck flask equipped with a stirrer, a thermometer, a cork-stoppered nitrogen gas inlet tube, and a cork-stoppered glass tube. While the mixture was stirred at 95°C, the moisture was dried off under reduced pressure. The inner pressure of the four-neck flask was returned back to the normal pressure by using a nitrogen gas. Then, 10.4 g of potassium carbonate serving as a catalyst (manufactured by Wako Pure Chemical Industries, Ltd., potassium carbonate) was added thereto, and the pressure was reduced again. The mixture was allowed to react by stirring at 110°C to 170°C for 45 hours under reduced pressure. After the completion of the reaction, the mixture was diluted with 1500 ml of xylene and filtered. The filtrate was slowly and repeatedly washed with warm water until the aqueous solution layer as a lower layer became substantially neutral. After the completion of the washing, the xylene layer as an upper layer was dried under reduced pressure, and then subjected to a decoloration treatment with activated carbon and activated clay. This was also subjected to a deodorization and distillation treatment by an ordinary method. By so doing, 733 g of the target trehalose fatty acid ester composition 8 having a hydroxyl value of 53 and a saponification value of 174 in a paste form was obtained.

### (Synthesis Example 9)

### Trehalose Fatty Acid Ester Composition 9 Obtained by Transesterifying Trehalose with Methyl Behenate

60.8 g (1 mole) of trehalose dihydrate (manufactured by Hayashibara Group., Treha powder), 439.2 g (7.7 moles) of methyl behenate, and 15.2 g of fatty acid sodium (manufactured by Miyoshi Oil & Fat Co., Ltd., TP-NA) were charged in a 1000 mL four-neck flask equipped with a stirrer, a thermometer, a cork-stoppered nitrogen gas inlet tube, and a cork-stoppered glass tube. While the mixture was stirred at 100°C, the moisture was dried off under reduced pressure. The inner pressure of the four-neck flask was returned back to the normal pressure by using a nitrogen gas. Then, 1.83 g of potassium carbonate serving as a catalyst (manufactured by Wako Pure Chemical Industries, Ltd., potassium carbonate) was added thereto, and the pressure was reduced again. The mixture was allowed to react by stirring at 110°C to 160°C for 45 hours under reduced pressure. After the completion of the reaction, the mixture was diluted with 100 g of xylene. The diluted matter was neutralized by adding warm water and 3.4 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 317 g of the target trehalose fatty acid ester composition 9 having a hydroxyl value of 28 and a saponification value of 170 was obtained.

### (Synthesis Example 10)

### Trehalose Fatty Acid Ester Composition 10 Obtained by Transesterifying Trehalose with Methyl Behenate

72.9 g (1 mole) of trehalose dihydrate (manufactured by Hayashibara Group., Treha powder), 427.1 g (6.25 moles) of methyl behenate, and 18.2 g of fatty acid sodium (manufactured by Miyoshi Oil & Fat Co., Ltd., TP-NA) were charged in a 1000 mL four-neck flask equipped with a stirrer, a thermometer, a cork-stoppered nitrogen gas inlet tube, and a cork-stoppered glass tube. While the mixture was stirred at 100°C, the moisture was dried off under reduced pressure. The inner pressure of the four-neck flask was returned back to the normal pressure by using a nitrogen gas. Then, 4.37 g of potassium carbonate serving as a catalyst (manufactured by Wako Pure Chemical Industries, Ltd., potassium carbonate) was added thereto, and the pressure was reduced again. The mixture was allowed to react by stirring at 110°C to 160°C for 45 hours under reduced pressure. After the completion of the reaction, the mixture was diluted with 100 g of xylene. The diluted matter was neutralized by adding warm water and 8.1 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 336 g of the target trehalose fatty acid ester composition 10 having a hydroxyl value of 68 and a saponification value of 167 was obtained.

### (Synthesis Example 11)

### Trehalose Fatty Acid Ester Composition 11 Obtained by Esterifying Trehalose with Stearic Acid Chloride

17.1 g (1 mole) of trehalose (manufactured by Wako Pure Chemical Industries, Ltd., trehalose anhydride) and pyridine were charged in a 500 mL four-neck flask equipped with a stirrer, a thermometer, a cork-stoppered nitrogen gas inlet tube, and a cork-stoppered dropping funnel, and stirred in an ice bath to be dissolved. 133.3 g (8.8 moles) of stearic acid chloride (manufactured by Tokyo Chemical Industry Co., Ltd., Stearoyl Chloride) was gradually added thereto. After the completion of the addition, the reaction container was taken out from the ice bath and the mixture was allowed to react by stirring at room temperature. After the completion of the reaction, the reaction container was placed back to the ice bath, and the mixture was diluted by adding 200 ml of diethyl ether. Thereafter, water was gradually added, and washing was slowly and repeatedly performed with water. After the completion of the washing, the diethyl ether layer as an upper layer was dried with magnesium sulfate. The magnesium sulfate was removed by filtration, and the diethyl ether in the filtrate was distilled off under reduced pressure. By so doing, 108 g of the target trehalose fatty acid ester composition 11 having a hydroxyl value of 0.3 and a saponification value of 179 was obtained.

### (Synthesis Example 12)

### Trehalose Fatty Acid Ester Composition 12 Obtained by Esterifying Trehalose with Palmitic Acid Chloride

17.1 g (1 mole) of trehalose (manufactured by Wako Pure Chemical Industries, Ltd., trehalose anhydride) and pyridine were charged in a 500 mL four-neck flask equipped with a stirrer, a thermometer, a cork-stoppered nitrogen gas inlet tube, and a cork-stoppered dropping funnel, and stirred in an ice bath to be dissolved. 120.9 g (8.8 moles) of palmitic acid chloride (manufactured by Wako Pure Chemical Industries, Ltd., palmitoyl chloride) was gradually added thereto. After the completion of the addition, the reaction container was taken out from the ice bath and the mixture was allowed to react by stirring at room temperature. After the completion of the reaction, the reaction container was placed back to the ice bath, and the mixture was diluted by adding 200 ml of diethyl ether. Thereafter, water was gradually added, and washing was slowly and repeatedly performed with water. After the completion of the washing, the diethyl ether layer as an upper layer was dried with magnesium sulfate. The magnesium sulfate was removed by filtration, and the diethyl ether in the filtrate was distilled off under reduced pressure. By so doing, 102 g of the target trehalose fatty acid ester composition 12 having a hydroxyl value of 0.5 and a saponification value of 195 was obtained.

### (Synthesis Example 13)

### Sucrose Fatty Acid Ester Composition 1 Obtained by Transesterifying Sucrose with Methyl Stearate

168.5 g (1 mole) of sucrose (sucrose manufactured by Wako Pure Chemical Industries, Ltd.), 113.1.5 g (7.7 moles) of methyl stearate (manufactured by Wako Pure Chemical Industries, Ltd., methyl stearate), and 33.7 g of fatty acid sodium (manufactured by Miyoshi Oil & Fat Co., Ltd., TP-NA) were charged in a 2000 mL four-neck flask equipped with a stirrer, a thermometer, a cork-stoppered nitrogen gas inlet tube, and a cork-stoppered glass tube. While the mixture was stirred at 100°C, the moisture was dried off under reduced pressure. The inner pressure of the four-neck flask was returned back to the normal pressure by using a nitrogen gas. Then, 5.7 g of potassium carbonate serving as a catalyst (manufactured by Wako Pure Chemical Industries, Ltd., potassium carbonate) was added thereto, and the pressure was reduced again. The mixture was allowed to react by stirring at 110°C to 160°C for 60 hours under reduced pressure. After the completion of the reaction, the mixture was diluted with 260 g of xylene. The diluted matter was neutralized by adding warm water and 5.7 g of citric acid anhydride (manufactured by Fuso Chemical Co., Ltd. citric acid (anhydride)). Thereafter, washing was slowly and repeatedly performed with warm water until the aqueous solution layer as a lower layer became substantially neutral. After the completion of the washing, the xylene layer as an upper layer was dried under reduced pressure, and then subjected to a decoloration treatment with activated carbon. This was also subjected to a deodorization and distillation treatment by an ordinary method. By so doing, 1058 g of the target sucrose fatty acid ester composition 1 having a hydroxyl value of 24 and a saponification value of 177 was obtained.

**[Table 1]**

| Table 1 Compositions oftrehalose fatty acid ester compositions | | | |
|---|---|---|---|
| Name of sample | Average esterification degree (calculated by hydroxyl value) (calculated by HPLC analysis) | Hydroxyl value | Saponification value |
| Trehalose fatty acid ester composition 1 | 6.7 | 35 | 174 |
| | 6.6 | | |
| Trehalose fatty acid ester composition 2 | 5.1 | 94 | 167 |
| | 5.4 | | |
| Trehalose fatty acid ester composition 3 | 7.0 | 28 | 180 |
| | 7.0 | | |
| Trehalose fatty acid ester composition 4 | 5.1 | 99 | 169 |
| | 5.4 | | |
| Trehalose fatty acid ester composition 5 | 7.2 | 22 | 195 |
| | 7.1 | | |
| Trehalose fatty acid ester composition 6 | 5.3 | 89 | 184 |
| | 5.4 | | |
| Trehalose fatty acid ester composition 7 | 3.2 | 242 | 160 |
| | 3.8 | | |
| Trehalose fatty acid ester composition 8 | 6.1 | 53 | 174 |
| | 6.2 | | |
| Trehalose fatty acid ester composition 9 | 6.8 | 28 | 150 |
| | 6.8 | | |
| Trehalose fatty acid ester composition 10 | 5.6 | 63 | 146 |
| | 5.9 | | |
| Trehalose fatty acid ester composition 11 | 7.99 | 0.3 | 179 |
| | 7.98 | | |
| Trehalose fatty acid ester composition 12 | 7.98 | 0.5 | 195 |
| | 7.98 | | |
| Sucrose fatty acid ester composition 1 | 7.0 | 24 | 177 |
| | 7.0 | | |

The results of the HPLC analysis (area percentage (% by area) of each ester) of the trehalose fatty acid ester compositions 1 to 12, the sucrose fatty acid ester composition 1, and the sucrose polystearate are shown in Table 2. In Table 2, the term "Raw materials" denotes the % by area of the remaining raw materials, the numbers "1" to "8" respectively denote the % by area of a monoester, a diester, a triester, a tetraester, a pentaester, a hexaester, a heptaester, and an octaester.

**[Table 2]**

| Table 2 | % by area of each ester as determined by HPLC analysis | | | | | | | | |
|---|---|---|---|---|---|---|---|---|---|
| | Raw materials | 1 | 2 | 3 | 4 | 5 | 6 | 7 | 8 |
| Trehalose fatty acid ester composition 1 | 0 | 0 | 0 | 1.0 | 3.2 | 11.2 | 25.0 | 39.5 | 20.1 |
| Trehalose fatty acid ester composition 2 | 0 | 0 | 3.4 | 8.7 | 16.0 | 23.4 | 22.8 | 16.8 | 8.9 |
| Trehalose fatty acid ester composition 3 | 0 | 0 | 0 | 0 | 0.0 | 6.1 | 18.3 | 36.6 | 39.0 |
| Trehalose fatty acid ester composition 4 | 0 | 0 | 2.0 | 8.8 | 16.2 | 24.8 | 23.8 | 17.1 | 7.3 |
| Trehalose fatty acid ester composition 5 | 0 | 0 | 0 | 0 | 0.0 | 5.6 | 19.4 | 38.5 | 36.5 |
| Trehalose fatty acid ester composition 6 | 0 | 0 | 0 | 8.5 | 16.3 | 20.8 | 24.1 | 20.1 | 10.2 |
| Trehalose fatty acid ester composition 7 | 0 | 0 | 16.7 | 28.8 | 27.5 | 17.2 | 7.5 | 2.1 | 0.2 |
| Trehalose fatty acid ester composition 8 | 0.7 | 0 | 0 | 1.6 | 9.3 | 17.8 | 24.6 | 27.5 | 18.5 |
| Trehalose fatty acid ester composition 9 | 0 | 0 | 0 | 0 | 0.3 | 8.2 | 25.1 | 41.2 | 25.2 |
| Trehalose fatty acid ester composition 10 | 0 | 0 | 0 | 4.5 | 14.8 | 17.4 | 28.2 | 24.7 | 10.4 |
| Trehalose fatty acid ester composition 11 | 0 | 0 | 0 | 0 | 0 | 0 | 0 | 0.3 | 99.7 |
| Trehalose fatty acid ester composition 12 | 0 | 0 | 0 | 0 | 0 | 0 | 0 | 1.7 | 98.3 |
| Sucrose fatty acid ester composition 1 | 0 | 0 | 0 | 1.2 | 1.2 | 5.6 | 17.3 | 35.7 | 39.0 |
| Sucrose polystearate | 2.1 | 0 | 3.7 | 7.9 | 17.4 | 29.8 | 27.7 | 11.4 | 0 |
| Composition of Example 6 | 0 | 0 | 0 | 0.5 | 1.6 | 5.6 | 12.5 | 19.9 | 59.9 |
| Composition of Comparative Example 10 | 0 | 0 | 6.7 | 11.5 | 11.0 | 6.9 | 3.0 | 1.0 | 59.9 |

### <Evaluation of hardness>

### (Method for preparing evaluation sample)

All the components were weighed at the blending amounts shown in Tables 3 to 20 in a container. The mixture was stirred at 100°C for 30 minutes to be heated, dissolved, and mixed. By so doing, a homogeneous mixture was prepared. The prepared mixture was poured in a PC cylindrical container (having an inner diameter of 37 mm and a height of 16 mm) and stored at 25°C for 24 hours. Thereafter, the product was used as a sample for the hardness evaluation. Regarding the components in the Tables, trehalose isostearate esters are Nomcort TQ-5 (manufactured by the Nisshin OilliO Group, Ltd.), sucrose polystearate is Cosmelike S-10 (manufactured by Dai-ichi Kogyo Seiyaku Co., Ltd.), sucrose tetrastearate triacetate is Cosmelike SA-10 (manufactured by Dai-ichi Kogyo Seiyaku Co., Ltd.), neopentyl glycol dicaprate is Estemol N-01 (manufactured by the Nisshin OilliO Group, Ltd.), and the paraffin wax is HNP-9 (manufactured by Nippon Seiro Co., Ltd.).

### (Evaluation method)

Each evaluation sample was measured for the maximum stress at the time when a stainless steel ball of Φ2.5 mm was inserted for 2.5 mm, by using a rheometer (manufactured by Sun. Scientific Co Ltd., Product name: RHEO TEX SD-700) (the speed of insertion: 60 mm/min). The evaluation results of the hardness are shown in FIG. 1 and Tables 3 to 21.

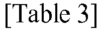

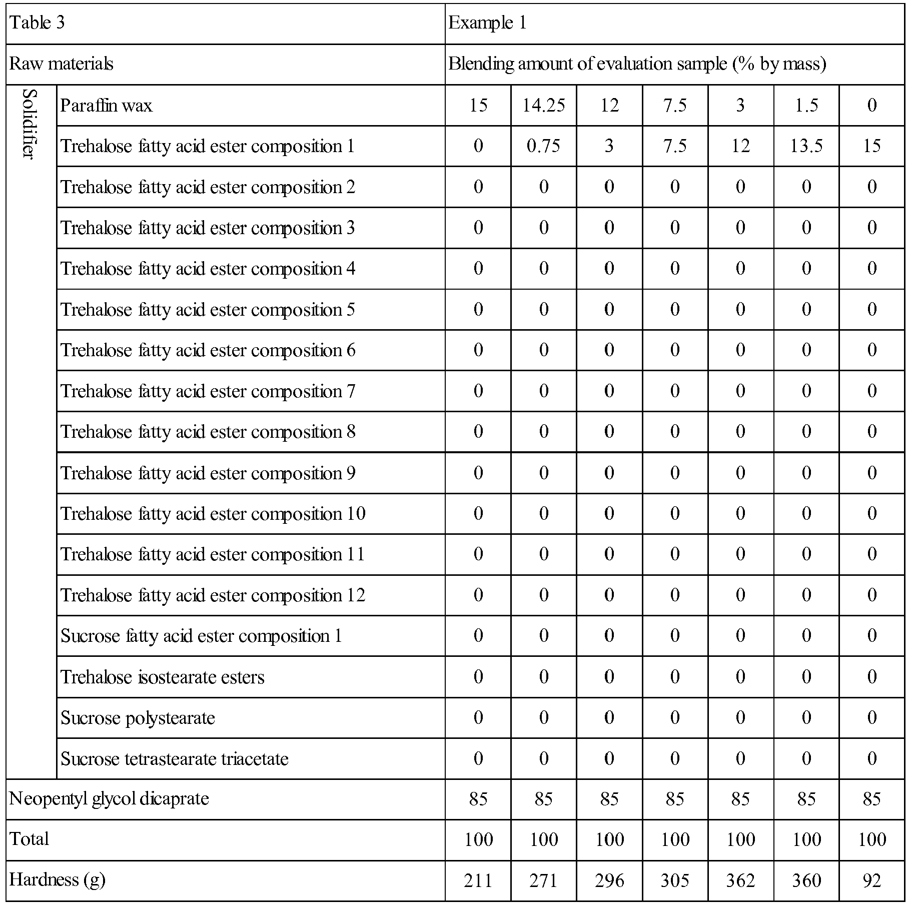

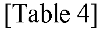

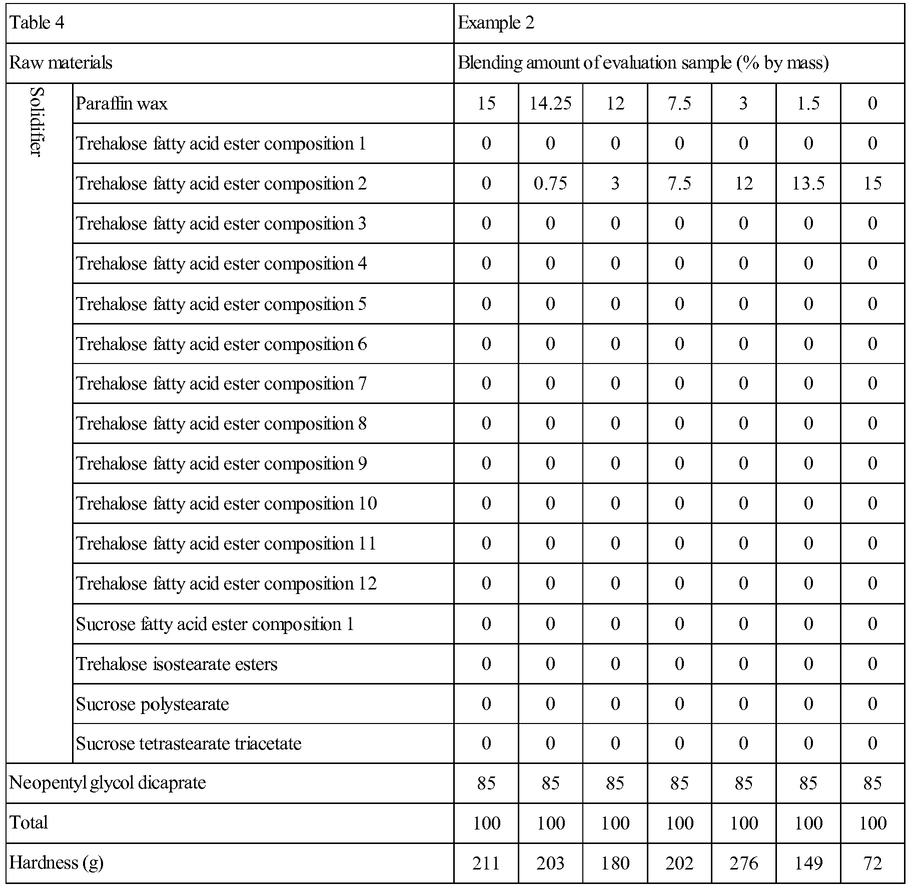

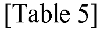

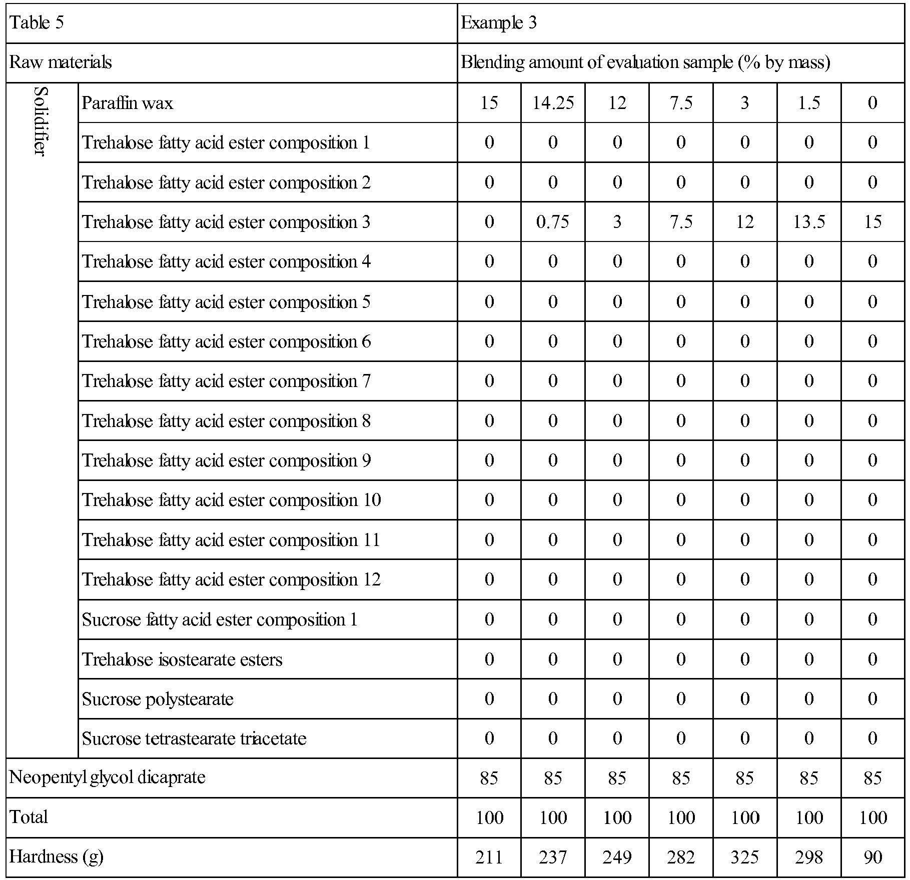

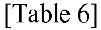

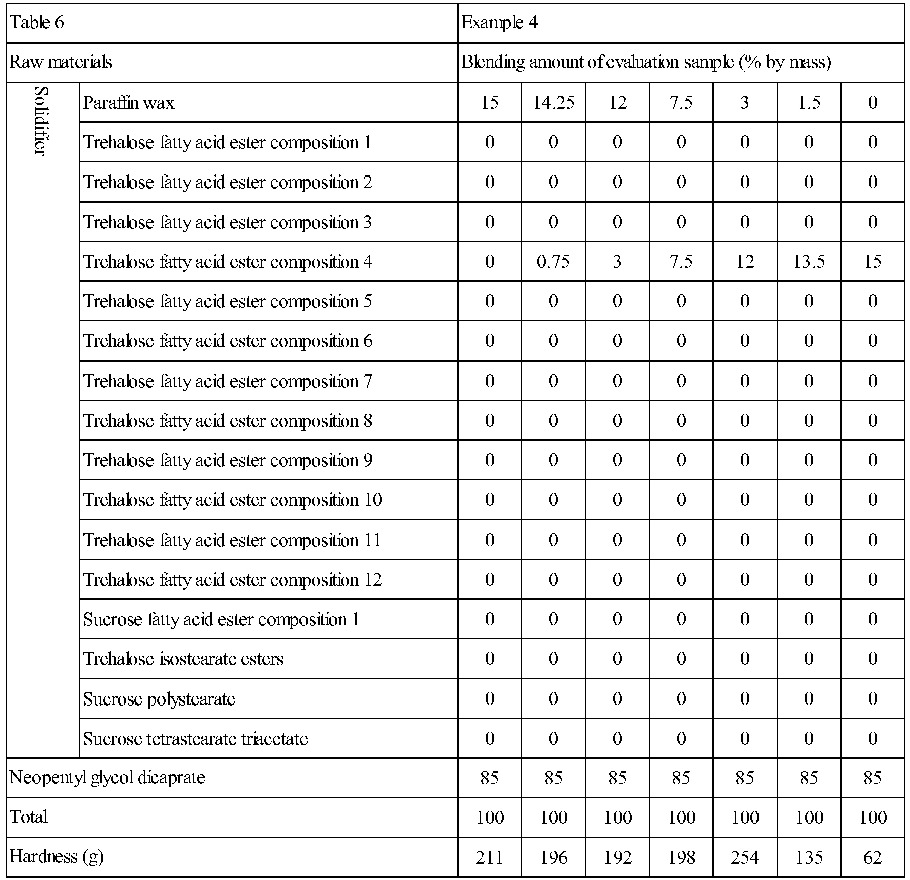

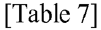

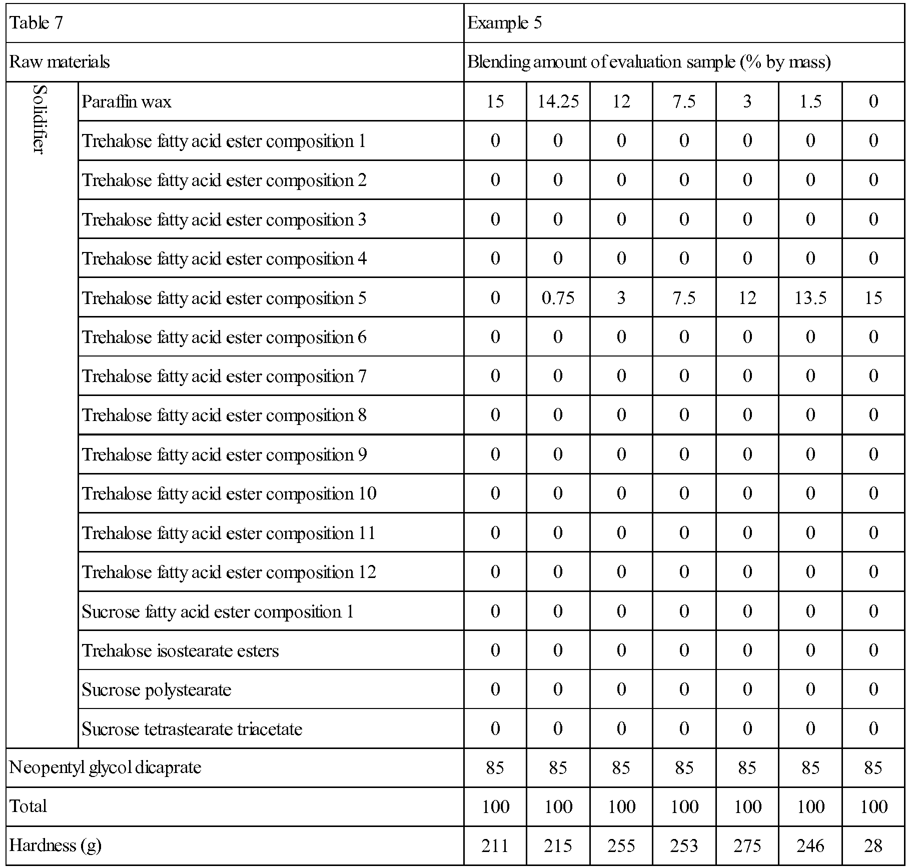

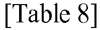

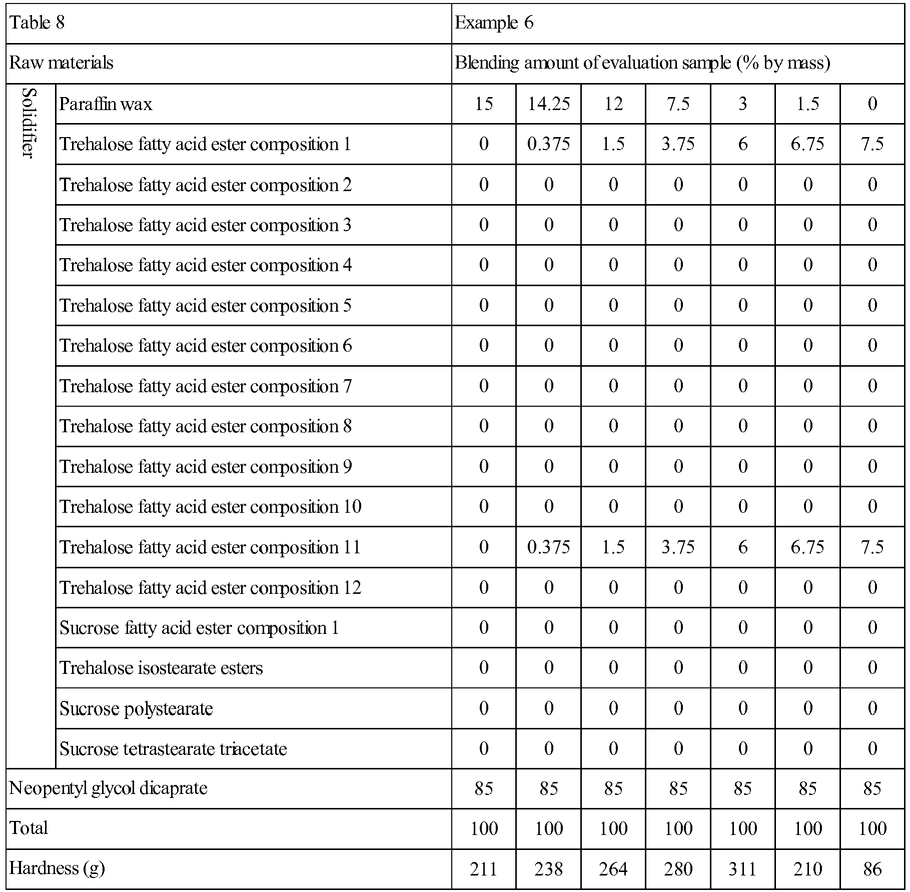

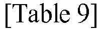

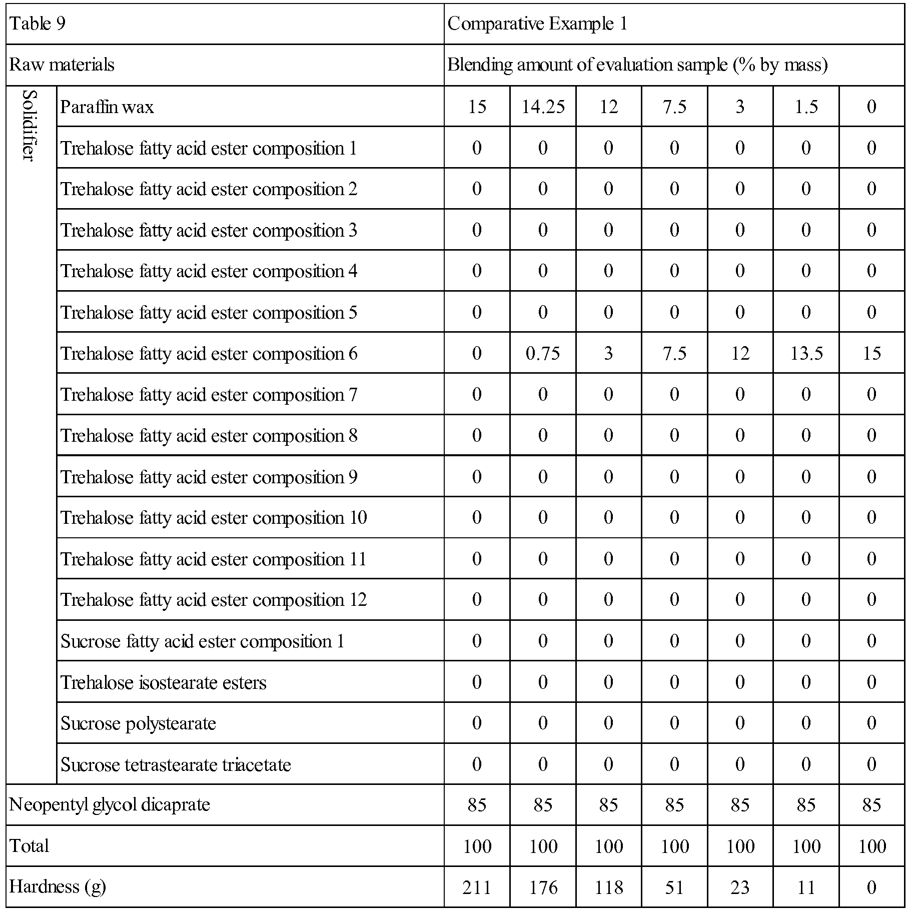

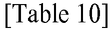

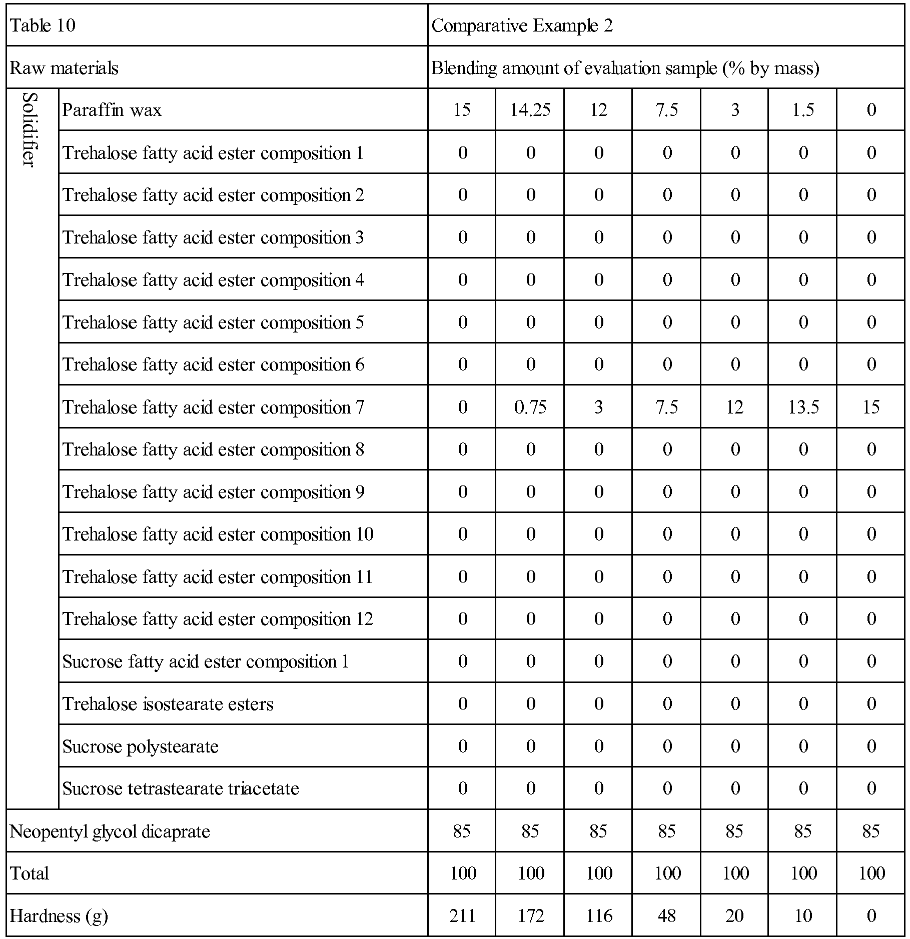

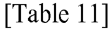

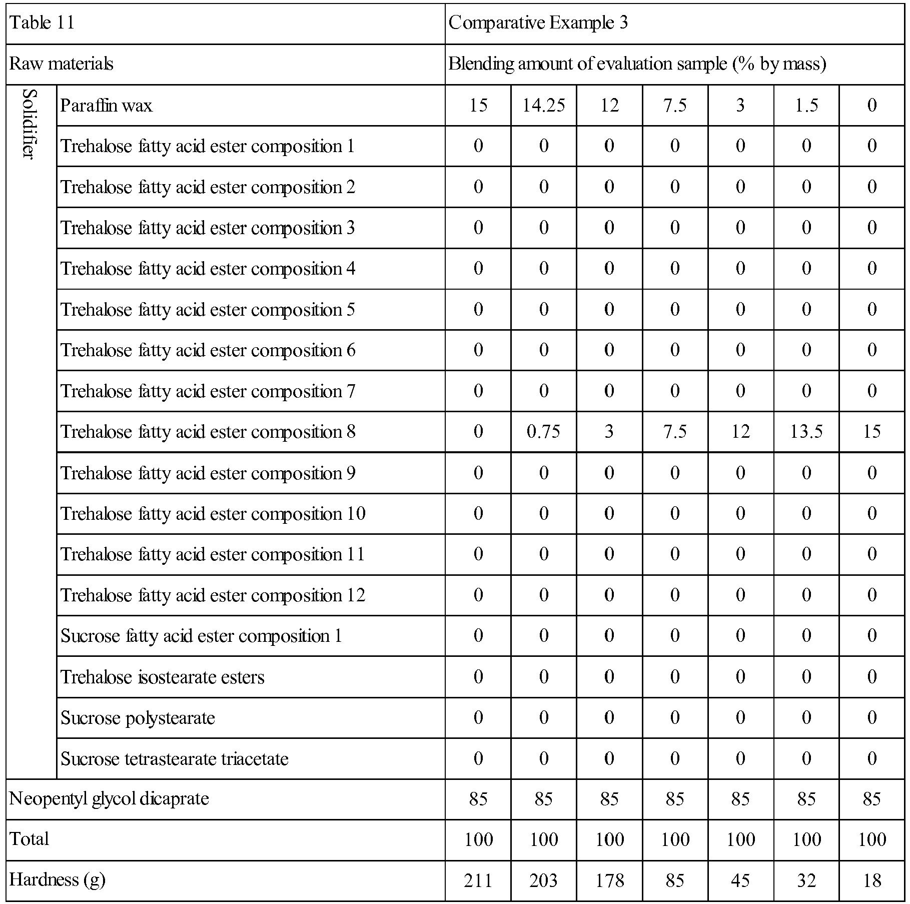

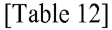

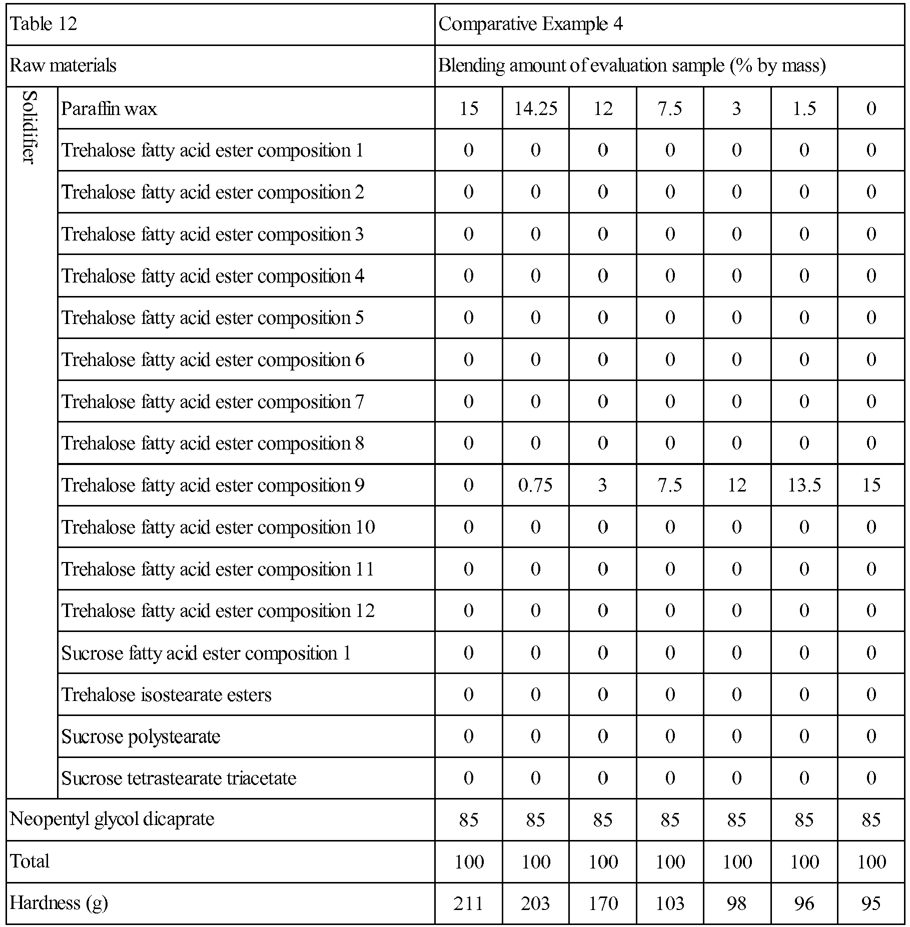

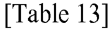

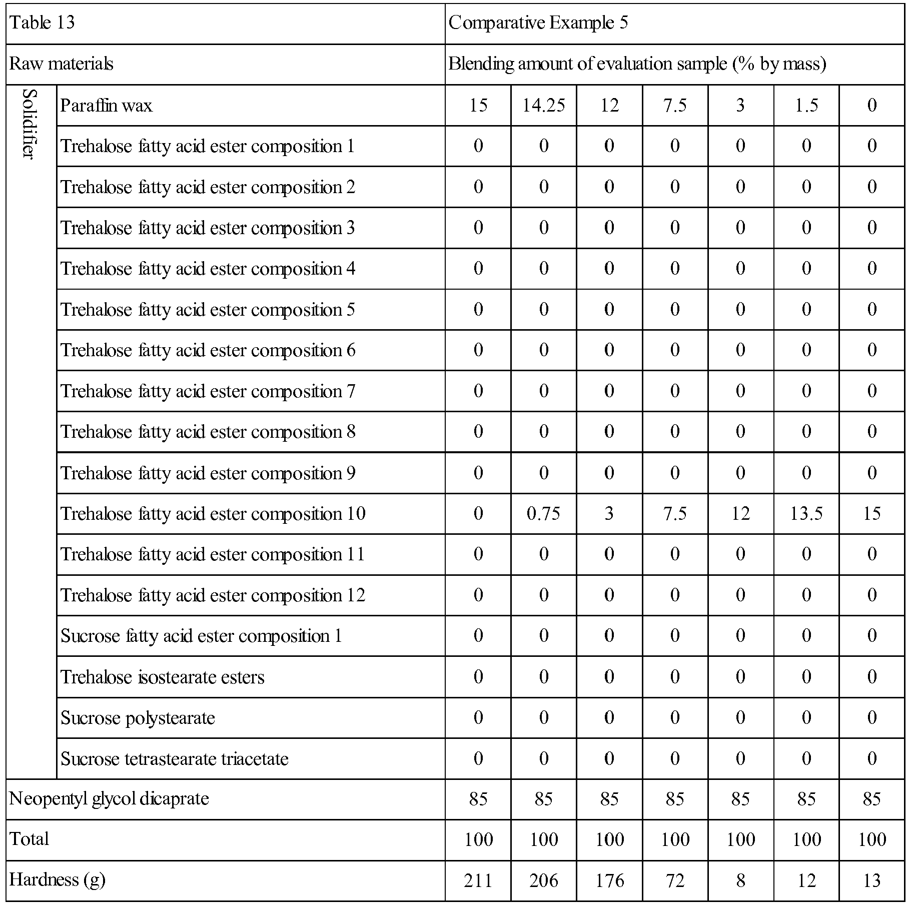

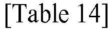

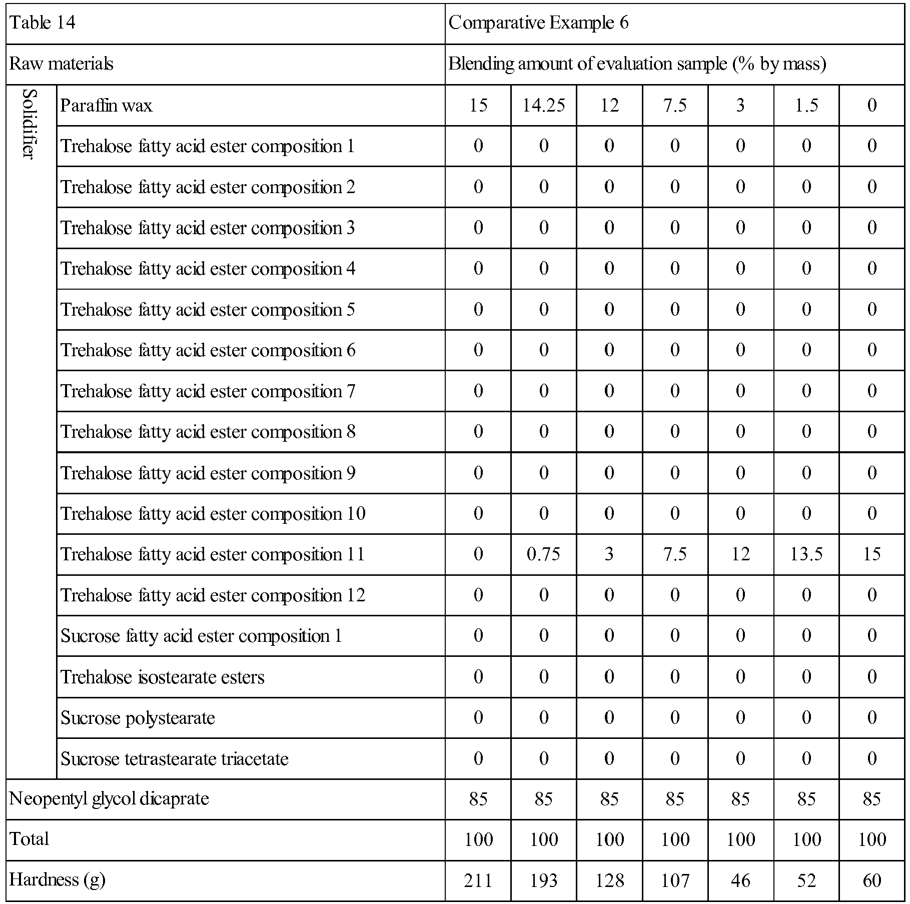

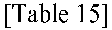

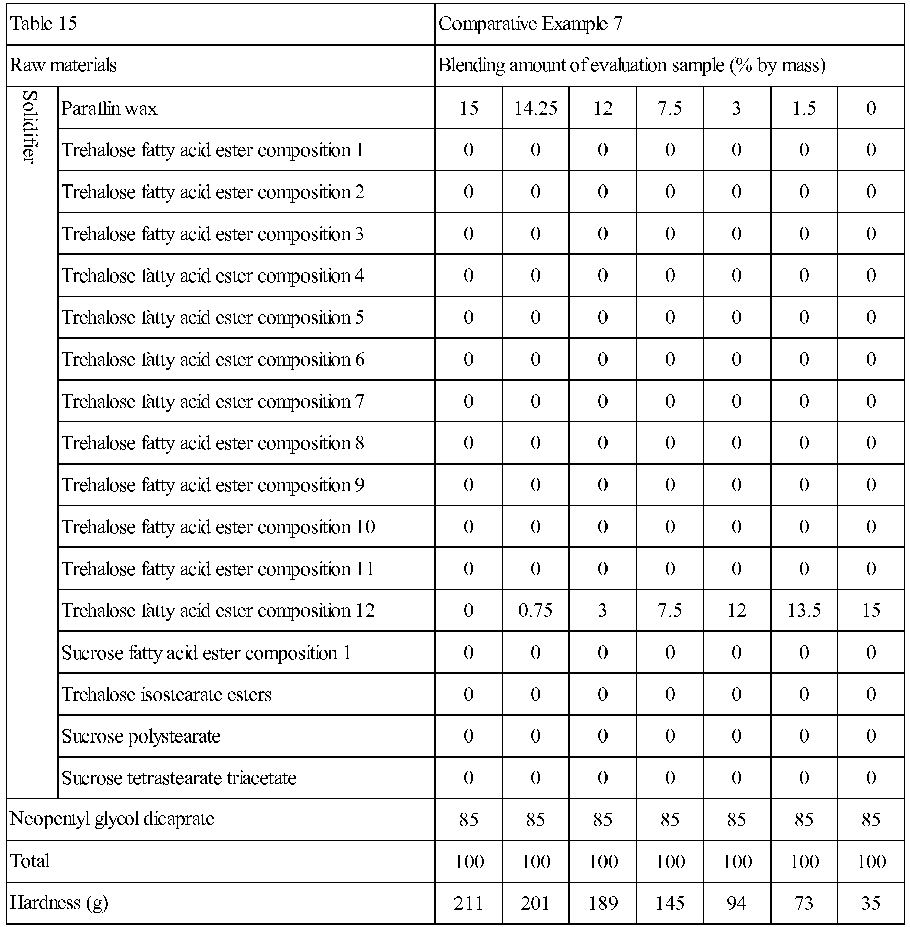

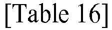

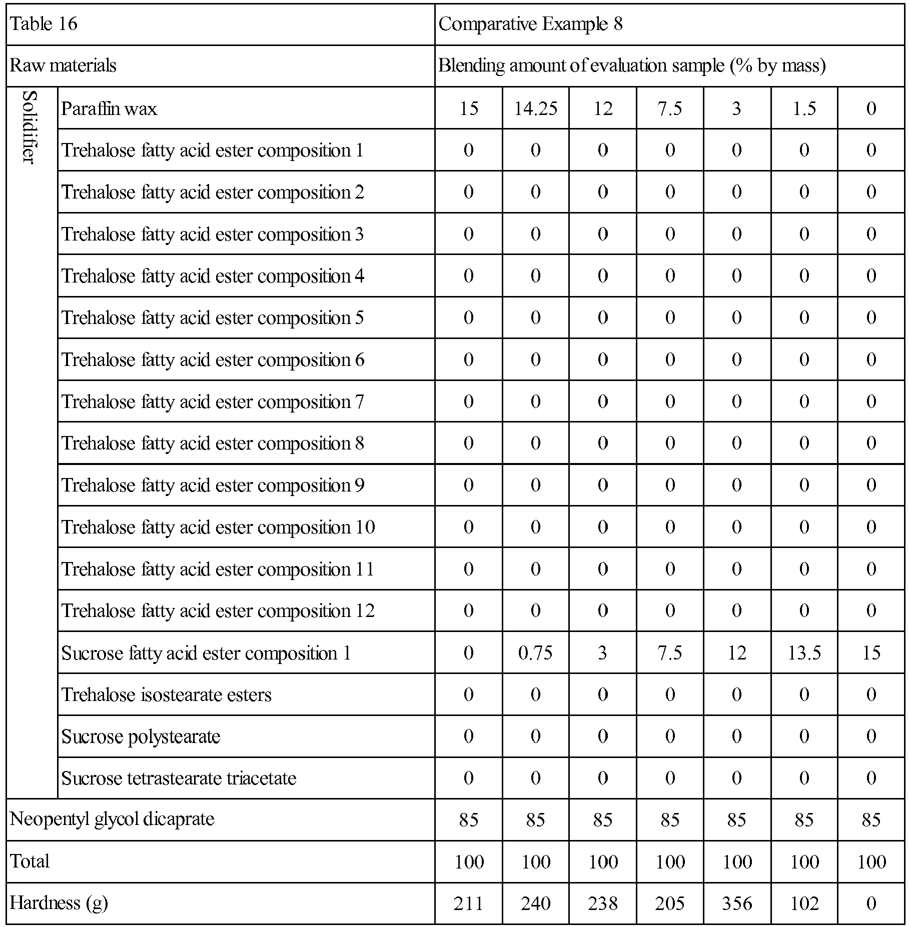

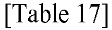

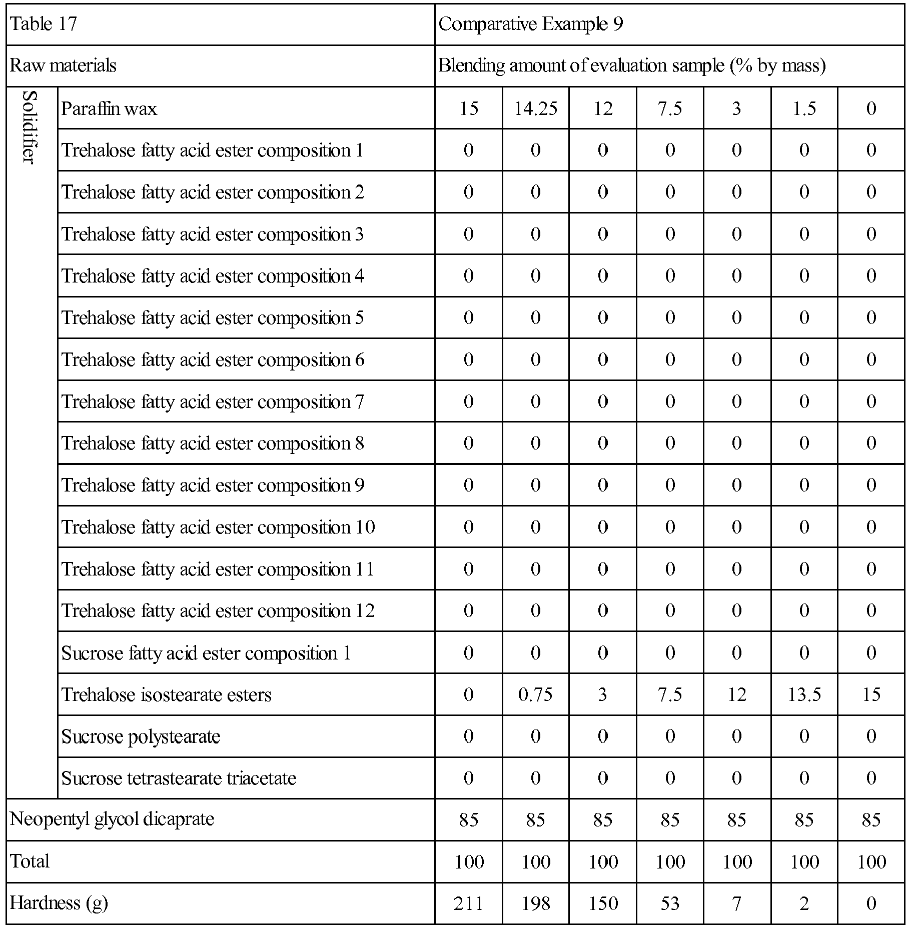

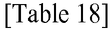

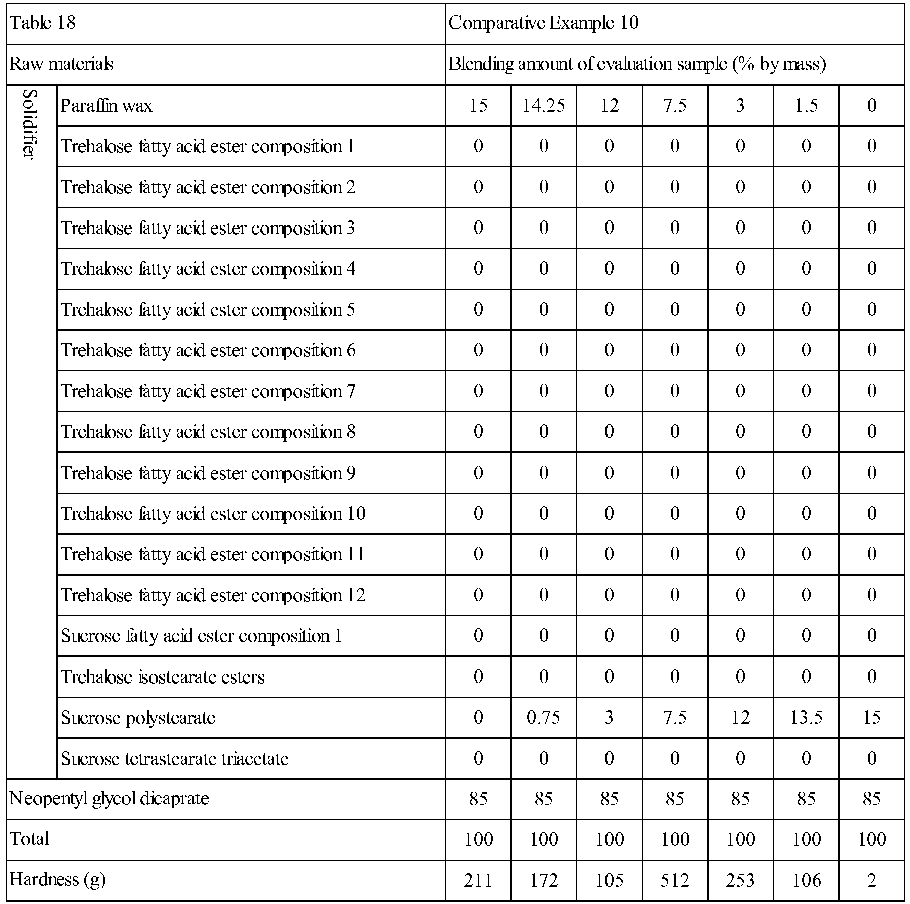

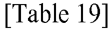

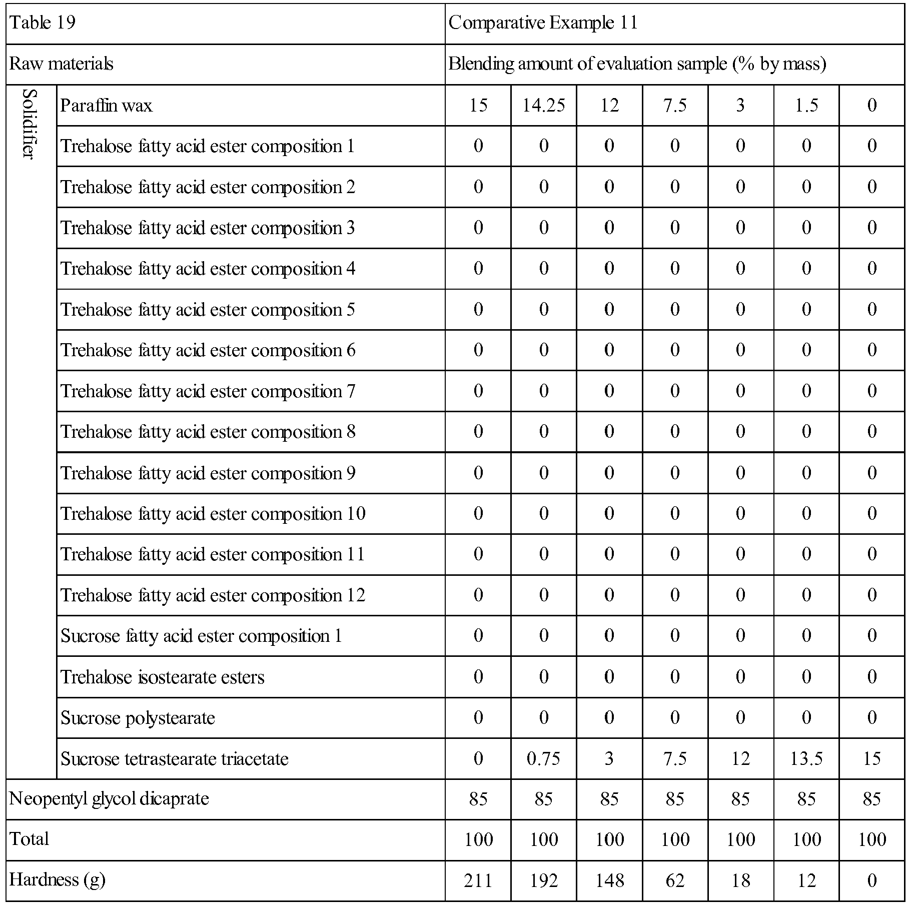

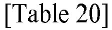

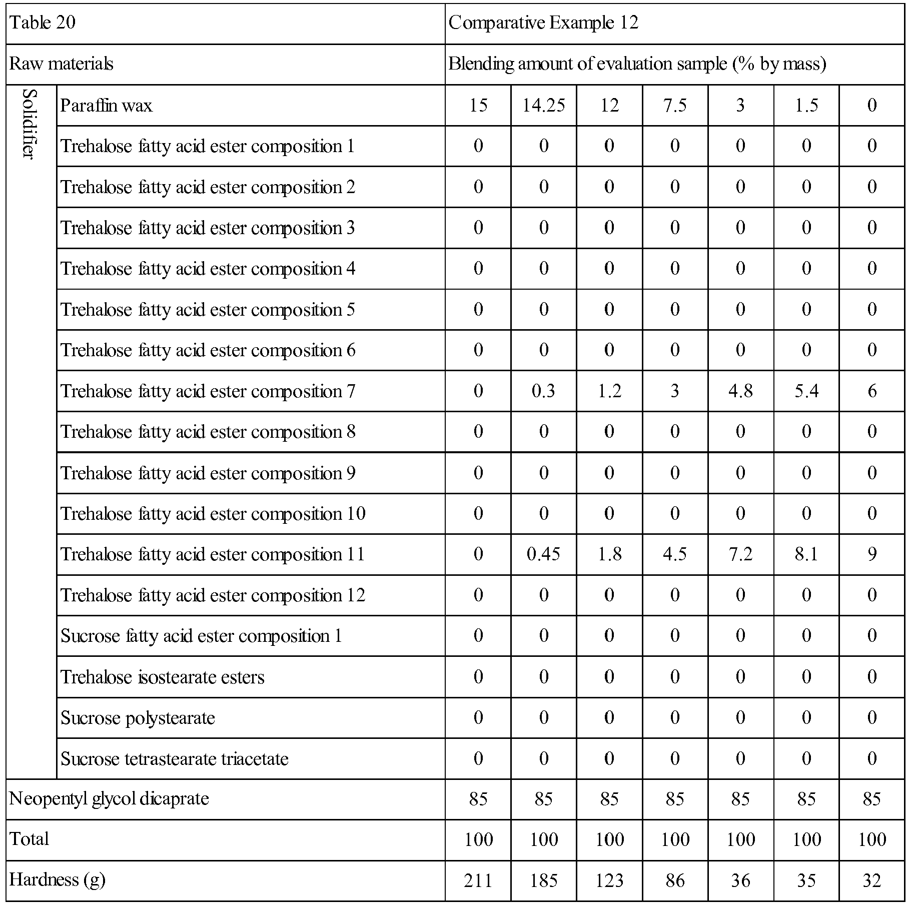

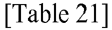

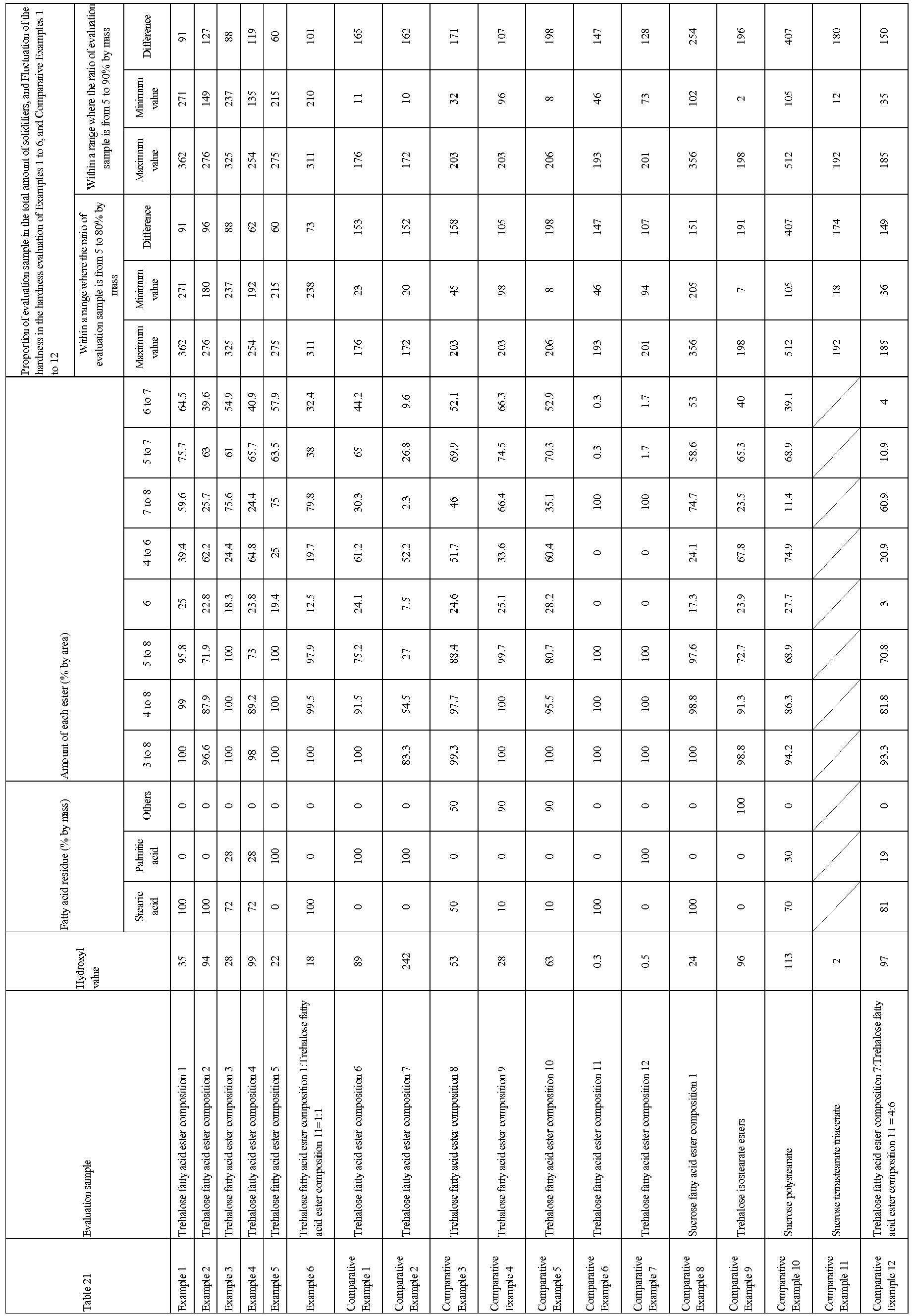

### (Evaluation results)

As is apparent from the results shown in FIG. 1 and Table 3 to Table 21, in Comparative Example 10 where sucrose polystearate, among from sucrose fatty acid ester compositions, had been blended, the hardness largely fluctuated due to the blending amount of the sucrose fatty acid ester. In other words, when a small amount (0.75 to 3% by mass) of sucrose polystearate was blended, the hardness was gradually decreased lower than the case without blending it. However, the hardness was rapidly increased when the amount was much increased, and the hardness was again rapidly decreased when the blending ratio was even more increased. On the other hand, in Comparative Example 11 where sucrose tetrastearate triacetate prepared by acetylating sucrose polystearate had been blended, the hardness was decreased as the blending amount of sucrose tetrastearate triacetate was increased. This confirmed that there was no effect of increasing the hardness by blending. Moreover, in Comparative Example 8 where the sucrose fatty acid ester composition 1 had been blended, although the phenomenon of the decrease and rapid increase of the hardness was slightly suppressed, the difference between the maximum value and the minimum value of the hardness was 151 when the blending amount was from 0.75 to 12% by mass (5 to 80% by mass relative to the total amount of solidifiers). This confirmed that the fluctuation of the hardness due to the blending amount was large.

Conversely, in Examples 1 to 5 where the trehalose fatty acid ester composition had been blended, the fluctuation of the hardness due to the blending amount of the trehalose fatty acid ester composition was small. When the composition was blended at 5% by mass relative to the total amount of solidifiers, the hardness was slightly increased. The hardness was slightly increased until the composition accounted for 80% by mass. From Table 21, in Examples 1 to 5, the difference between the maximum value and the minimum value of the hardness was 100 or smaller when the blending amount was from 0.75 to 12% by mass (5 to 80% by mass relative to the total amount of solidifiers). Also from this, the fluctuation of the hardness can be said to be small. Furthermore, in Examples 1,3, and 5, the difference between the maximum value and the minimum value of the hardness was 100 or smaller when the blending amount was from 0.75 to 15% by mass (5 to 90% by mass relative to the total amount of solidifiers). This confirmed that the fluctuation of the hardness is small even though the blending amount was much increased.

In addition, the hydroxyl value of the trehalose fatty acid ester composition of Example 6 obtained by the calculation from the blending ratios of the trehalose fatty acid ester compositions 1 and 11 was 18, and the difference between the maximum value and the minimum value of the hardness was 73 when the blending amount of the trehalose fatty acid ester compositions 1 and 11 was from 0.75 to 12% by mass (5 to 80% by mass relative to the total amount of solidifiers). This confirmed that the fluctuation of the hardness due to the blending amount was small.

On the other hand, in Comparative Examples 1 to 7 and 9 where the trehalose fatty acid ester composition differing from the trehalose fatty acid ester composition of the present invention had been blended, no increase of the hardness was seen irrespective of the blending amount.

Moreover, the hydroxyl value of the trehalose fatty acid ester composition of Comparative Example 12 obtained by the calculation from the blending ratios of the trehalose fatty acid ester compositions 7 and 11 was 97, which is within the range of the aspect of the present invention. However, the sum amount of a tetraester, a pentaester, and a heptaester, relative to the total peak area, calculated by HPLC analysis was 20.9% by area, and furthermore, the sum amount of a hexaester and a heptaester, relative to the total peak area, calculated by HPLC analysis was 4.0% by area, which are out of the range of the aspect of the present invention. The difference between the maximum value and the minimum value of the hardness was 149 when the blending amount was from 0.75 to 12% by mass (5 to 80% by mass relative to the total amount of solidifiers). This confirmed that the fluctuation of the hardness due to the blending amount was large.

### <Evaluation of thermostability>

### (Method for preparing evaluation sample)

20 g of the evaluation sample shown in Table 22 and neopentyl glycol dicaprate (manufactured by the Nisshin OilliO Group, Ltd., Product name: Estemol N-01) were weighed. The mixture was heated, dissolved, and mixed homogeneously at 100°C. By so doing, a homogeneous mixture was prepared. The mixture at 100°C prepared by the above-mentioned method was poured in a stoppered sample vial of Φ26 mm. This was used as an evaluation sample for the thermostability test.

### (Evaluation method)

The sample prepared by the above-mentioned method was left to stand in a thermostat bath at 120°C to perform the thermostability test. The changes in the color tone and the odor after 24 hours were checked and evaluated according to the following evaluation criteria.

[Change in color tone]
A: No change
B: Slight change is seen.
C: Significant change is seen.
[Change in odor]
A: No change
B: Slight deterioration odor is smelt.
C: Significant deterioration odor is smelt.

**[Table 22]**

| Table 22 | Evaluation sample | Evaluation results of color tone | Evaluation results of odor |
|---|---|---|---|
| Example 7 | Trehalose fatty acid ester composition 1 | A | A |
| Example 8 | Trehalose fatty acid ester composition 2 | A | A |
| Example 9 | Trehalose fatty acid ester composition 3 | A | A |
| Example 10 | Trehalose fatty acid ester composition 4 | A | A |
| Example 11 | Trehalose fatty acid ester composition 5 | A | A |
| Example 12 | Trehalose fatty acid ester composition 6 | A | A |
| Example 13 | Trehalose fatty acid ester composition 7 | B | B |
| Example 14 | Trehalose fatty acid ester composition 8 | A | A |
| Example 15 | Trehalose fatty acid ester composition 9 | A | A |
| Example 16 | Trehalose fatty acid ester composition 10 | A | A |
| Example 17 | Trehalose fatty acid ester composition 11 | A | A |
| Example 18 | Trehalose fatty acid ester composition 12 | A | A |
| Example 19 | Trehalose isostearate esters | A | A |
| Comparative Example 13 | Sucrose fatty acid ester composition 1 | B | B |
| Comparative Example 14 | Sucrose polystearate | C | C |
| Comparative Example 15 | Sucrose tetrastearate triacetate | B | B |

The evaluation results of changes in the color tone and the odor are shown in Table 22. As a result, the trehalose fatty acid ester compositions showed smaller changes or almost no change in both the color tone and the odor, as compared to those of sucrose polystearate, and thus it was found that these compositions were superior in the thermostability. In Comparative Example 14 where sucrose polystearate had been blended, a brown burn mark was found at the bottom part after cooling and solidification.

### <Evaluation of lip cream>

### (Evaluation sample)

All the components were weighed at the blending amounts shown in Table 23 or Table 24. The mixture was heated and dissolved at 100°C, and then well mixed. The obtained mixture was kept at 80°C to be defoamed. After the defoaming, the mixture was poured and filled in a mold. This was cooled down to room temperature to be molded. The molded solid matter was taken out from the mold and put in a container. By so doing, a lip cream was obtained.

Regarding the components in the tables, dipentaerythritol hexaoxystearate is COSMOL 168M (manufactured by the Nisshin OilliO Group, Ltd.), diisostearyl malate is COSMOL 222 (manufactured by the Nisshin OilliO Group, Ltd.), polyglyceryl diisostearate is COSMOL 42V (manufactured by the Nisshin OilliO Group, Ltd.), and polyglyceryl triisostearate is COSMOL 43V (manufactured by the Nisshin OilliO Group, Ltd.).

In addition, the blending amounts of the respective components of Examples 20 to 28 shown in Table 24 were adjusted so that the hardness of the produced lip cream would be equivalent to that of a commercial product.

**[Table 23]**

| Table 23 | | |
|---|---|---|
| Component | Raw materials | Blending amount (parts by weight) |
| A | Candelillla wax | 3 |
| | Dipentaerythritol hexaoxystearate | 10 |
| | Diisostearyl malate | 20 |
| | Squalane | 20 |
| | Polyglyceryl diisostearate | 12 |
| | Polyglyceryl triisostearate | 12 |
| | Hydrogenated polybutene | 8 |

**[Table 24]**

| Table 24 | Blending amount of lip cream (% by mass) | | | | | | | | | | |
|---|---|---|---|---|---|---|---|---|---|---|---|
| Raw materials | Examples | | | | | | | | | Comparative Examples | |
| | 20 | 21 | 22 | 23 | 24 | 25 | 26 | 27 | 28 | 16 | 17 |
| Paraffin wax | 17.6 | 11 | 4.4 | 20 | 11.5 | 5 | 20 | 11.5 | 4.4 | 20 | 5 |
| Trehalose fatty acid ester composition 2 | 4.4 | 11 | 17.6 | 0 | 0 | 0 | 0 | 0 | 0 | 0 | 0 |
| Trehalose fatty acid ester composition 4 | 0 | 0 | 0 | 5 | 11.5 | 20 | 0 | 0 | 0 | 0 | 0 |
| Trehalose fatty acid ester composition 5 | 0 | 0 | 0 | 0 | 0 | 0 | 5 | 11.5 | 17.6 | 0 | 0 |
| Sucrose polystearate | 0 | 0 | 0 | 0 | 0 | 0 | 0 | 0 | 0 | 5 | 20 |
| Component A | 78 | 78 | 78 | 75 | 77 | 75 | 75 | 77 | 78 | 75 | 75 |
| Total | 100 | 100 | 100 | 100 | 100 | 100 | 100 | 100 | 100 | 100 | 100 |

### (Evaluation method)

30 women that had used make-up for 10 years or longer were employed as the evaluation panelists and the sensory evaluation was conducted on the obtained lip creams based on a 5 point rating scale (5: Excellent, 4: Very good, 3: Good, 2: Fair, 1: Poor) for each item of "smooth spreading ability", "attachability", "glossiness" and "stickiness".

### (Evaluation results)

The evaluation results of the lip creams are shown in Table 25. As a result, in Comparative Examples 16 and 17 where sucrose polystearate serving as a sucrose fatty acid ester had been blended, a large difference was found in the hardness, meaning that the sucrose fatty acid ester showed inferior results to the trehalose fatty acid ester composition also in the sensory evaluation. In other words, the sucrose fatty acid ester was revealed to have many problems such as a deterioration in the sense of use at the time of application, because the hardness of the cosmetic blended therewith largely fluctuated due to the blending amount.

On the other hand, in Examples 20 to 28 where the trehalose fatty acid ester composition of the present invention had been blended, the hardness did not fluctuate so largely even though the blending amount of the trehalose fatty acid ester composition had been changed. Furthermore, in the sensory evaluation, those having a greater blending amount of the trehalose fatty acid ester composition showed better evaluation results.

**[Table 25]**

| Table 25 | Hardness (g) | Smooth spreading ability | Attachability | Glossiness | Stickiness | Total |
|---|---|---|---|---|---|---|
| Example 20 | 604 | 2 | 2 | 3 | 4 | 11 |
| Example 21 | 526 | 4 | 3 | 3 | 4 | 14 |
| Example 22 | 563 | 5 | 4 | 4 | 4 | 17 |
| Example 23 | 553 | 3 | 2 | 2 | 4 | 11 |
| Example 24 | 578 | 4 | 3 | 4 | 4 | 15 |
| Example 25 | 586 | 5 | 4 | 4 | 5 | 18 |
| Example 26 | 593 | 3 | 3 | 2 | 3 | 11 |
| Example 27 | 547 | 3 | 4 | 3 | 4 | 14 |
| Example 28 | 586 | 4 | 5 | 4 | 4 | 17 |
| Comparative Example 16 | 721 | 1 | 1 | 2 | 3 | 7 |
| Comparative Example 17 | 351 | 3 | 3 | 2 | 4 | 12 |
| Commercial product | 593 | 3 | 4 | 3 | 3 | 13 |

### <Evaluation of rouge>

### (Evaluation sample)

All the components were weighed at the blending amounts shown in Table 26. The mixture was heated and dissolved at 120°C, and then well mixed. The obtained mixture was kept at 80°C to be defoamed. After the defoaming, the mixture was poured and filled in a mold. This was cooled down to room temperature to be molded. The molded solid matter was taken out from the mold and put in a container. By so doing, a stick rouge was obtained.

Regarding the components in the table, diisostearyl malate is COSMOL 222 (manufactured by the Nisshin OilliO Group, Ltd.), cetyl 2-ethylhexanoate is SALACOS 816T (manufactured by the Nisshin OilliO Group, Ltd.), hexa(hydroxy stearic acid / stearic acid / rosin acid) dipentaerythrityl is COSMOL 168ARV (manufactured by the Nisshin OilliO Group, Ltd.), hydrogenated polybutene is NOMCORT HP-30 (manufactured by the Nisshin OilliO Group, Ltd.), and triethylhexanoin is T.I.O (manufactured by the Nisshin OilliO Group, Ltd.).

**[Table 26]**

| Table 26 | Blending amount of rouge (% by mass) | | | | | | | |
|---|---|---|---|---|---|---|---|---|
| Raw materials | Examples | | Comparative Examples | | | | | |
| | 29 | 30 | 18 | 19 | 20 | 21 | 22 | 23 |
| Polyethylene wax | 6 | 5 | 6 | 5 | 6 | 5 | 6 | 5 |
| Candelilla wax | 8 | 4 | 8 | 4 | 8 | 4 | 8 | 4 |
| Trehalose fatty acid ester composition 1 | 5 | 10 | 0 | 0 | 0 | 0 | 0 | 0 |
| Sucrose fatty acid ester composition 1 | 0 | 0 | 5 | 10 | 0 | 0 | 0 | 0 |
| Sucrose polystearate | 0 | 0 | 0 | 0 | 5 | 10 | 0 | 0 |
| Microcrystalline wax | 0 | 0 | 0 | 0 | 0 | 0 | 5 | 10 |
| Diisostearyl malate | 20 | 20 | 20 | 20 | 20 | 20 | 20 | 20 |
| Cetyl 2-ethylhexanoate | 17 | 17 | 17 | 17 | 17 | 17 | 17 | 17 |
| Hexa(hydroxy stearic acid / stearic acid / rosin acid) dipentaerythrityl | 13 | 13 | 13 | 13 | 13 | 13 | 13 | 13 |
| Hydrogenated polybutene | 6 | 6 | 6 | 6 | 6 | 6 | 6 | 6 |
| Triethylhexanoin | 20 | 20 | 20 | 20 | 20 | 20 | 20 | 20 |
| Titanium oxide | 3 | 3 | 3 | 3 | 3 | 3 | 3 | 3 |
| Mica | 0.5 | 0.5 | 0.5 | 0.5 | 0.5 | 0.5 | 0.5 | 0.5 |
| Red No. 201 | 1 | 1 | 1 | 1 | 1 | 1 | 1 | 1 |
| Red No. 202 | 0.5 | 0.5 | 0.5 | 0.5 | 0.5 | 0.5 | 0.5 | 0.5 |
| Total | 100 | 100 | 100 | 100 | 100 | 100 | 100 | 100 |

### (Method of "sensory evaluation")

30 women that had used make-up for 10 years or longer were employed as the evaluation panelists. They were allowed to use the above-mentioned rouges of Examples 25 and 26 and Comparative Examples 18 and 19. Then, the sensory evaluation was conducted based on a 5 point rating scale (5: Excellent, 4: Very good, 3: Good, 2: Fair, 1: Poor) for each item of "smooth spreading ability", "attachability", "glossiness" and "stickiness". In addition, the rouges of Comparative Examples 16 and 17 were broken at the time of the application, and thus the evaluation was not feasible.

### (Evaluation method regarding "shape retainability, oil stains, and stability over time")

The obtained stick rouges were evaluated by observing the shape retainability at the time of application and oil stains. The shape retainability at the time of application was evaluated according to the evaluation criteria of Table 27.

Moreover, the obtained stick rouges were stored in thermostat baths at temperatures of 40°C, 50°C, and between 20°C and 40°C. The changes in the appearance for up to 1 month were observed, and the stability over time was evaluated according to the evaluation criteria of Table 28.

**[Table 27]**

| Table 27 Evaluation criteria for shape retainability | |
|---|---|
| Observation of behavior when applied to the skin | Evaluation Scores |
| Able to be applied without a problem | A |
| Slightly soft feeling but within an unproblematic range | B |
| Soft feeling but able to be applied | C |
| Broken at the time of application and unable to be applied | D |

**[Table 28]**

| Table 28 Evaluation criteria for stability over time | |
|---|---|
| Observation of changes in appearance for up to 1 month after storage in a thermostat bath at temperatures of 40°C, 50°C, and between 20°C and 40°C | Evaluation Scores |
| No change in appearance | A |
| Slight change in appearance but within an unproblematic range | B |
| Change in appearance beyond the acceptable range | C |
| Not only changes in appearance but also other serious changes such as breakage | D |

### (Evaluation results)

The evaluation results of the stick rouges are shown in Table 29. As a result, in Comparative Examples 18 to 21 where sucrose polystearate or the sucrose fatty acid ester composition 1 serving as a sucrose fatty acid ester had been blended, inferior results, for example, a breakage of the stick rouge after one week at 50°C, to those of the trehalose fatty acid ester composition, were seen in the evaluation of the stability over time. In other words, the sucrose fatty acid ester was revealed to have many problems when blended in a rouge, because it was difficult to retain the shape of the blended cosmetic, and the stability over time was inferior.

In addition, in Comparative Examples 22 and 23 where microcrystalline wax for general use in a rouge had been blended, the stability over time was almost equivalent to that of the trehalose fatty acid ester composition. However, inferior results to those of the trehalose fatty acid ester composition were seen in the sensory evaluation, because the smooth spreading ability and the attachability were inferior.

On the other hand, in Examples 29 and 30 where the trehalose fatty acid ester composition of the present invention had been blended, there was no problem in the shape retainability and the stability over time even though the blending amount of the trehalose fatty acid ester composition had been changed. Furthermore, in the sensory evaluation, those having a greater blending amount of the trehalose fatty acid ester composition showed better evaluation results.

**[Table 29]**

| Table 29 | Evaluation results of stick rouge | | | | | | | |
|---|---|---|---|---|---|---|---|---|
| | Examples | | Comparative Examples | | | | | |
| | 29 | 30 | 18 | 19 | 20 | 21 | 22 | 23 |
| Hardness | 680 | 604 | 150 | 73 | 614 | 538 | 451 | 314 |
| Evaluation item | Evaluation results | | | | | | | |
| Smooth spreading ability | 5 | 5 | - | - | 3 | 4 | 1 | 2 |
| Attachability | 3 | 4 | - | - | 2 | 3 | 1 | 3 |
| Glossiness | 3 | 4 | - | - | 2 | 2 | 1 | 3 |
| Stickiness | 3 | 3 | - | - | 3 | 2 | 2 | 1 |
| Shape retainability | A | A | D | D | B | B | A | A |
| Oil stains | Not found | Not found | Not found | Not found | Found | Found | Not found | Not found |
| Stability at 40°C | A | A | B | B | B | B | B | B |
| Stability at 50°C | A | A | D | D | D | D | B | B |
| Stability between 20 and 40°C | A | A | B | B | B | B | A | A |

### <Evaluation of stick concealer>

### (Evaluation sample)

All the components were weighed at the blending amounts shown in Table 30. The mixture was heated and dissolved at 100°C, and then well mixed. The obtained mixture was kept at 80°C to be defoamed. After the defoaming, the mixture was poured and filled in a container. This was then cooled down to room temperature. By so doing, a stick concealer was obtained.

The obtained stick concealer was excellent in the shape retainability and the stability over time, had no stickiness, an excellent shielding effect, and satisfactory make-up lasting.

**[Table 30]**

| Table 30 Stick concealer | |
|---|---|
| Raw materials | % by mass |
| Trehalose fatty acid ester composition 1 | 5 |
| Polyethylene wax | 4 |
| Ceresin | 3 |
| Paraffin wax | 6 |
| Isononyl isononanoate | 32.7 |
| Polybutene | 5 |
| Dimethyl polysiloxane | 3 |
| Trehalose isostearate esters | 5 |
| Titanium oxide | 20 |
| Red iron oxide | 0.8 |
| Yellow iron oxide | 5 |
| Black iron oxide | 0.5 |
| Talc | 10 |
| Total | 100 |

### <Evaluation of eye-color pencil>

### (Evaluation sample)

All the components were weighed at the blending amounts shown in Table 31. The mixture was heated and dissolved at 85°C, and then well mixed. The obtained mixture was kept at 80°C to be defoamed. After the defoaming, the mixture was poured into a shift hole on the back end side of a cylindrical shift made from a resin and filled therein. This was then cooled down to be solidified. By so doing, an eye-color pencil was obtained.

The obtained eye-color pencil was excellent in the shape retainability and the stability over time, with glossiness and satisfactory make-up finishing.

**[Table 31]**

| Table 31 Eye-color pencil | |
|---|---|
| Raw materials | % by mass |
| Trehalose fatty acid ester composition 1 | 5 |
| Ceresin | 6 |
| Candelilla wax | 4 |
| Bee wax | 5 |
| Macadamia nut oil | 10.4 |
| Trehalose isostearate esters | 30 |
| Diisostearyl malate | 7 |
| Natural vitamin E | 0.1 |
| Mica | 3 |
| Cobalt blue | 1.5 |
| Mica titanium | 28 |
| Total | 100 |

<Evaluation of clay wax>

### (Evaluation sample)

All the components were weighed at the blending amounts shown in Table 32. The mixture was heated and dissolved at 80°C, and then well mixed. The obtained mixture was kept at 80°C, and the deformed mixture was poured and filled in a container. This was then cooled down. By so doing, a clay wax was obtained.

The obtained clay wax was excellent in the stability over time, had no stickiness, and a satisfactory hair-setting property.

**[Table 32]**

| Table 32 Clay wax | |
|---|---|
| Raw materials | % by mass |
| Liquid paraffin | 53.3 |
| Trehalose fatty acid ester composition 2 | 5 |
| Vaseline | 7 |
| Talc | 30 |
| Quaternium-18 hectorite | 0.5 |
| Trehalose isostearate esters | 2 |
| Candelilla wax | 2 |
| Propyl paraoxybenzoate | 0.09 |
| N atural vitamin E | 0.1 |
| Perfume | 0.01 |
| Total | 100 |

### <Evaluation of oil type foundation>

### (Evaluation sample)

All the components were weighed at the blending amounts shown in Table 33. The mixture was heated at 80°C, and then dispersed homogeneously. The obtained mixture was poured and filled in a container at 80°C. This was cooled down. By so doing, an oil type foundation was obtained.

The obtained oil type foundation was excellent in the shape retainability and the stability over time, was able to be smoothly spread, and offered a favorable sense of application with a moist rich feeling and satisfactory make-up lasting.

**[Table 33]**

| Table 33 Oil type foundation | |
|---|---|
| Raw materials | % by mass |
| Trehalose fatty acid ester composition 2 | 2 |
| Hydrogenated polydecene | 17 |
| Ethylhexyl palmitate | 9.5 |
| Trehalose isostearate esters | 1 |
| Phenyl trimethicone | 4.5 |
| Dimethicone | 14.75 |
| Paraffin | 6 |
| Titanium oxide | 9.52 |
| Talc | 3.06 |
| Iron oxide | 1.02 |
| Mica | 21.5 |
| Polymethyl methacrylate | 10 |
| Tocopherol | 0.05 |
| Butylparaben | 0.05 |
| Methylparaben | 0.05 |
| Total | 100 |

### <Evaluation of moist cream (O/W)>

### (Evaluation sample)

The component A and the component B were respectively weighed at the blending amounts shown in Table 34, and heated at 70°C. The component B was gradually added while the component A was being mixed by a dispersion mixer. The obtained mixture was cooled down to 25°C. By so doing, a moist cream was obtained.

The obtained moist cream was excellent in the stability over time, was able to be smoothly spread, and offered a favorable sense of application with a moist rich feeling and excellent lasting of the moist rich feeling.

**[Table 34]**

| Table 34 Moist cream (O/W) | | |
|---|---|---|
| | Raw materials | % by mass |
| A | Purified water | 53.84 |
| | Glycerin | 12 |
| | BG | 6 |
| | Ethylparaben | 0.1 |
| | Propylparaben | 0.05 |
| | EDTA-2Na | 0.01 |
| | Glyceryl stearate | 3 |
| | PEG-100 stearate | 2 |
| | Cetanol | 1.5 |
| B | Behenyl alcohol | 2.5 |
| | Trehalose fatty acid ester composition 3 | 1 |
| | Mineral oil | 5 |
| | Diisostearyl malate | 4 |
| | Cetyl ethylhexanoate | 2 |
| | Triethylhexanoin | 2 |
| | Dimethicone | 5 |
| | Total | 100 |

### <Evaluation of eye liner>

### (Evaluation sample)

All the components were weighed at the blending amounts shown in Table 35. The mixture was heated at 90°C, and then dispersed homogeneously. The obtained mixture was poured and filled in a container at 80°C. This was cooled down. By so doing, an eye liner was obtained.

The obtained eye liner was excellent in the stability over time, was able to be smoothly spread, and offered satisfactory make-up finishing.

**[Table 35]**

| Table 35 Eye liner | |
|---|---|
| Raw materials | % by mass |
| Carnauba wax | 5 |
| Trehalose fatty acid ester composition 2 | 5 |
| Microcrystalline wax | 6 |
| White vaseline | 1 |
| Liquid polyisobutylene | 72.5 |
| Organic bentonite | 0.5 |
| Titanium oxide | 8 |
| Carbon black | 2 |
| Total | 100 |

### <Evaluation of mascara>

The component B and a part of the component D were mixed at the blending amounts shown in Table 36, and dispersed by three rollers to become a paste. The component A and a part of the component D were heated to 90°C, and stirred to be homogeneous. The former paste was added thereto. The mixture was heated to 80°C or less while being stirred at high speed. A mixture of the component C and the remaining part of the component D having been stirred to be homogeneous, was added thereto. The mixture was heated to 90°C while being stirred, and then cooled down to 30°C. By so doing, a mascara was obtained.

The obtained mascara was excellent in the stability over time, and showed excellent adhesion.

**[Table 36]**

| Table 36 Mascara | | |
|---|---|---|
| | Raw materials | % by mass |
| A | Carnauba wax | 7.9 |
| | Candelilla wax | 0.5 |
| | Polyethylene wax | 5 |
| | Trehalose fatty acid ester composition 3 | 3 |
| B | Sorbitan monolaurate | 0.2 |
| | Black iron oxide | 1.5 |
| | Ultramarine blue | 9 |
| | Red iron oxide | 0.1 |
| C | Aluminum stearate | 1.8 |
| D | Epoxy resin isostearate ester | 12 |
| | Epoxy resin stearate ester | 2 |
| | Volatile isoparaffin | 53 |
| | Volatile silicone | 4 |
| | Total | 100 |

### <Evaluation of stick eye shadow>

The component A at the blending amounts shown in Table 37 was heated and dissolved at 90°C. Then, the mixed component B was added thereto. The mixture was dispersed by three rollers to become a paste. This was again dissolved and defoamed. Then, this was poured in a mold and cooled down to 25°C. By so doing, a stick eye shadow was obtained.

The obtained stick eye shadow was excellent in the shape retainability and the stability over time, with satisfactory glossiness and satisfactory make-up finishing.

**[Table 37]**

| Table 37 Stick eye shadow | | |
|---|---|---|
| | Raw materials | % by mass |
| A | Trehalose fatty acid ester composition 1 | 4 |
| | Ceresin | 3 |
| | Candelilla wax | 2 |
| | Glyceryl tri(2-ethylhexanoate) | 34 |
| | Sorbitan sesquioleate | 3 |
| B | Mica titanium | 34 |
| | Nylon powder | 8.5 |
| | Titanium oxide | 4 |
| | Red iron oxide | 1 |
| | Ultramarine blue | 6.5 |
| | Total | 100 |

### <Evaluation of hair styling wax (O/W)>

The component A and the component B were respectively weighed at the blending amounts shown in Table 38, and heated and dissolved at 95°C. The component B was gradually added while the component A was being stirred by a paddle so that the mixture was emulsified. The mixture was cooled down to 50°C while being stirred by a paddle, and the component C was added thereto. By so doing, a hair styling wax was obtained.

The obtained hair styling wax was excellent in the stability over time, and offered satisfactory hair styling ability and long lasting holding ability.

**[Table 38]**

| Table 38 Hair styling wax (O/W) | | |
|---|---|---|
| | Raw materials | % by mass |
| A | Polyethylene | 8 |
| | Carnauba wax | 5 |
| | Mineral oil | 5 |
| | Paraffin | 4.5 |
| | Trehalose fatty acid ester composition 1 | 4.5 |
| | Trehalose fatty acid ester composition 9 | 2 |
| | Dimethicone (10CS) | 4 |
| | Myristyl alcohol | 3.5 |
| | Cetyl ethylhexanoate | 3.5 |
| | PEG-30 glyceryl diisostearate | 2.5 |
| | Glyceryl stearate | 2.5 |
| | Stearic acid | 2.3 |
| | Ethylhexyl salicylate | 0.1 |
| | Phenoxyethanol | 0.1 |
| | Steareth-25 | 1.5 |
| B | Butylene glycol | 7 |
| | Methylparaben | 0.1 |
| | Propylparaben | 0.1 |
| | 0 | 0.1 |
| | Purified water | 43.6 |
| C | Perfume | 0.1 |
| | Total | 100 |

### <Evaluation of cleansing cream (O/W)>

The component A and the component B were respectively weighed at the blending amounts shown in Table 39, and heated at 75°C. The component B was gradually added while the component A was being mixed by a homomixer. to be emulsified. Then, the mixture was cooled down to 40°C while being stirred by a paddle. By so doing, a cleansing cream was obtained.

The obtained cleansing cream was excellent in the stability over time, had no stickiness, and satisfactorily melted with cosmetics.

**[Table 39]**

| Table 39 Cleansing cream (O/W) | | |
|---|---|---|
| | Raw materials | % by mass |
| A | PEG-30 glyceryl trioleate | 2 |
| | Trehalose fatty acid ester composition 4 | 1 |
| | Polyglyceryl-10 stearate | 1 |
| | Triethylhexanoin | 40 |
| | Cyclomethicone | 5 |
| | Cetanol | 3.5 |
| B | Butylene glycol | 8 |
| | Carbomer | 0.1 |
| | Sodium hydroxide | 0.1 |
| | Purified water | 39.3 |
| | Total | 100 |

### <Evaluation of lip gloss>

All the components were weighed at the blending amounts shown in Table 40. The mixture was heated and dissolved at 80°C, and then well mixed. The obtained mixture was poured and filled in a container at 80°C. This was cooled down. By so doing, a lip gloss was obtained.

The obtained a lip gloss was excellent in the stability over time, had glossiness, and offered satisfactory make-up finishing.

**[Table 40]**

| Table 40 Lip gloss | |
|---|---|
| Raw materials | % by mass |
| Trehalose fatty acid ester composition 5 | 25 |
| Castor oil | 70 |
| Dipentaerythrityl pentaisostearate | 5 |
| Total | 100 |

### <Evaluation of UV care cake foundation (W/O)>

The component A and the component B were respectively weighed at the blending amounts shown in Table 41, and heated and dissolved at 80°C. The component B was gradually added while the component A was being mixed by a homomixer so that the mixture was emulsified. The mixture was cooled down to 60°C while being stirred by a paddle. By so doing, a UV care cake foundation was obtained.

The obtained UV care cake foundation was excellent in the stability over time, had no stickiness, and offered satisfactory make-up lasting.

**[Table 41]**

| Table 41 UV care cake foundation (W/O) | | |
|---|---|---|
| | Raw materials | % by mass |
| A | Cyclopentasiloxane | 30 |
| | Ethylhexyl methoxycinnamate | 1 |
| | Candelilla wax | 2.5 |
| | Trehalose fatty acid ester composition 1 | 3 |
| | Paraffin wax | 3 |
| | Silica | 14 |
| | Zinc oxide | 1.5 |
| | Sorbitan isostearate | 4 |
| | Sorbitan tristearate | 4 |
| | Dimethicone (10cs) | 8 |
| | Dimethicone (350cs) | 3 |
| | Tocopherol acetate | 0.1 |
| | Propylparaben | 0.1 |
| B | Methylparaben | 0.1 |
| | Butylene glycol | 3 |
| | Sodium chloride | 0.2 |
| | Purified water | 22.5 |
| | Total | 100 |

### <Evaluation of vanishing cream (O/W)>

### (Evaluation sample)

The component A and the component B were respectively weighed at the blending amounts shown in Table 42, and heated at 80°C. The component B was slowly added while the component A was being mixed by a homodispersion mixer. The obtained mixture was cooled down to 25°C. By so doing, a vanishing cream was obtained.

The obtained vanishing cream was excellent in the stability over time, had no stickiness, and offered excellent sense of application.

**[Table 42]**

| Table 42 Vanishing cream (O/W) | | |
|---|---|---|
| | Raw materials | % by mass |
| A | Trehalose fatty acid ester composition 2 | 5 |
| | Sodium stearate | 14 |
| | Polyoxyethylene sorbitan monostearate | 2 |
| B | Propylene glycol | 14 |
| | Butylene glycol | 10 |
| | Glycerin | 6 |
| | Methylparaben | 0.2 |
| | Ethylparaben | 0.1 |
| | Propylparaben | 0.2 |
| | Purified water | 48.5 |
| | Total | 100 |

### <Evaluation of cold cream (O/W)>

### (Evaluation sample)

The component A was weighed at the blending amounts shown in Table 43, and heated at 85°C. The component B was slowly added while the component A was being mixed by a homomixer. The obtained mixture was cooled down to 25°C. By so doing, a cold cream was obtained.

The obtained cold cream was excellent in the stability over time, was able to be smoothly spread, and offered a favorable sense of application with a moist rich feeling and excellent lasting of the moist rich feeling.

**[Table 43]**

| Table 43 Cold cream (O/W) | | |
|---|---|---|
| | Raw materials | % by mass |
| A | Trehalose fatty acid ester composition 2 | 10 |
| | Paraffin wax | 5 |
| | Hexa(hydroxy stearic acid / stearic acid / rosin acid) dipentaerythrityl | 5 |
| | Vaseline | 5 |
| | Liquid paraffin | 10 |
| | Hydrogenated polyisobutene | 10 |
| | Isopropyl myristate | 12 |
| | Sorbitan sesquioleate | 2 |
| | Polyoxyethylene sorbitan monostearate | 1 |
| B | Purified water | 40 |
| | Total | 100 |

### INDUSTRIAL APPLICABILITY

The trehalose fatty acid ester of the present invention, and a wax composition including the same, can be suitably used particularly in the field of the production of solid cosmetics.

| Table 21 | Evaluation sample | Hydroxyl value | Fatty acid residue (% by mass) | | | Amount of each ester (% by area) | | | | | | | | Proportion of evaluation sample in the total amount of solidifiers, and Fluctuation of the hardness in the hardness evaluation of Examples 1 to 6, and Comparative Examples 1 to 12 | | | | | |
|---|---|---|---|---|---|---|---|---|---|---|---|---|---|---|---|---|---|---|---|
| | | | | | | | | | | | | | | Within a range where the ratio of evaluation sample is from 5 to 80% by mass | | | Within a range where the ratio of evaluation sample is from 5 to 90% by mass | | |
| | | | Stearic acid | Palmitic acid | Others | 3 to 8 | 4 to 8 | 5 to 8 | 6 | 4 to 6 | 7 to 8 | 5 to 7 | 6 to 7 | Max* value | Minx** value | Diff*** | Max* value | Min** value | Diff*** |
| Example 1 | TFAEC 1 | 35 | 100 | 0 | 0 | 100 | 99 | 95.8 | 25 | 39.4 | 59.6 | 73.7 | 64.5 | 362 | 271 | 91 | 362 | 271 | 91 |
| Example 2 | TFAEC 2 | 94 | 100 | 0 | 0 | 96.6 | 87.9 | 71.9 | 22.8 | 62.2 | 25.7 | 63 | 39.6 | 276 | 180 | 96 | 276 | 149 | 127 |
| Example 3 | TFAEC 3 | 28 | 72 | 28 | 0 | 100 | 100 | 100 | 18.3 | 24.4 | 75.6 | 61 | 54.9 | 325 | 237 | 88 | 325 | 237 | 88 |
| Example 4 | TFAEC 4 | 99 | 72 | 28 | 0 | 98 | 89.2 | 73 | 23.8 | 64.8 | 24.4 | 65.7 | 40.9 | 254 | 192 | 62 | 254 | 135 | 119 |
| Example 5 | TFAEC 5 | 22 | 0 | 100 | 0 | 100 | 100 | 100 | 19.4 | 25 | 75 | 63.5 | 57.9 | 275 | 215 | 60 | 275 | 215 | 60 |
| Example 6 | TFAEC 1 : TFAEC 11 = 1:1 | 18 | 100 | 0 | 0 | 100 | 99.5 | 97.9 | 12.5 | 19.7 | 79.8 | 38 | 32.4 | 311 | 238 | 73 | 311 | 210 | 101 |
| Comparative Example 1 | TFAEC 6 | 89 | 0 | 100 | 0 | 100 | 91.5 | 75.2 | 24.1 | 61.2 | 30.3 | 65 | 44.2 | 176 | 23 | 153 | 176 | 11 | 165 |
| Comparative Example 2 | TFAEC 7 | 242 | 0 | 100 | 0 | 83.3 | 54.5 | 27 | 7.5 | 52.2 | 2.3 | 26.8 | 9.6 | 172 | 20 | 152 | 172 | 10 | 162 |
| Comparative Example 3 | TFAEC 8 | 53 | 50 | 0 | 50 | 99.3 | 97.7 | 88.4 | 24.6 | 51.7 | 46 | 69.9 | 52.1 | 203 | 45 | 158 | 203 | 32 | 171 |
| Comparative Example 4 | TFAEC 9 | 28 | 10 | 0 | 90 | 100 | 100 | 99.7 | 25.1 | 33.6 | 66.4 | 74.5 | 66.3 | 203 | 98 | 105 | 203 | 96 | 107 |
| Comparative Example 5 | TFAEC 10 | 63 | 10 | 0 | 90 | 100 | 95.5 | 80.7 | 28.2 | 60.4 | 35.1 | 70.3 | 52.9 | 206 | 8 | 198 | 206 | 8 | 198 |
| Comparative Example 6 | TFAEC 11 | 0.3 | 100 | 0 | 0 | 100 | 100 | 100 | 0 | 0 | 100 | 0.3 | 0.3 | 193 | 46 | 147 | 193 | 46 | 147 |
| Comparative Example 7 | TFAEC 12 | 0.5 | 0 | 100 | 0 | 100 | 100 | 100 | 0 | 0 | 100 | 1.7 | 1.7 | 201 | 94 | 107 | 201 | 73 | 128 |
| Comparative Example 8 | Sucrose fatty acid ester composition 1 | 24 | 100 | 0 | 0 | 100 | 98.8 | 97.6 | 17.3 | 24.1 | 74.7 | 58.6 | 53 | 356 | 205 | 151 | 356 | 102 | 254 |
| Comparative Example 9 | Trehalose isostearate esters | 96 | 0 | 0 | 100 | 98.8 | 91.3 | 72.7 | 23.9 | 67.8 | 23.5 | 65.3 | 40 | 198 | 7 | 191 | 198 | 2 | 196 |
| Comparative Example 10 | Sucrose polystearate | 113 | 70 | 30 | 0 | 94.2 | 86.3 | 68.9 | 27.7 | 74.9 | 11.4 | 68.9 | 39.1 | 512 | 105 | 407 | 512 | 105 | 407 |
| Comparative Example 11 | Sucrose tetrastearate triacetate | 2 | | | | | | | | | | | | 192 | 18 | 174 | 192 | 12 | 180 |
| Comparative Example 12 | TFAEC 7 : TFAEC 11 = 4:6 | 97 | 81 | 19 | 0 | 93.3 | 81.8 | 70.8 | 3 | 20.9 | 60.9 | 10.9 | 4 | 185 | 36 | 149 | 185 | 35 | 150 |

| | | | | | | | | | | | | | | | | | | | |
|---|---|---|---|---|---|---|---|---|---|---|---|---|---|---|---|---|---|---|---|
| * Max = Maximum ** Min = Minimum *** Diff = Difference TFAEC: Trehalose fatty acid ester composition | | | | | | | | | | | | | | | | | | | |

## Claims

1. A trehalose fatty acid ester composition, comprising a trehalose fatty acid ester, wherein
all the fatty acid residues held by all the trehalose fatty acid esters in the composition are linear saturated fatty acid residues having 8 to 22 carbon atoms,
the composition comprises at least two types of esters selected from a group consisting of a triester, a tetraester, a pentaester, a hexaester, a heptaester, and an octaester, and
a sum amount of the esters relative to a total peak area, calculated by high performance liquid chromatography analysis is from 20 to 100% by area.

2. The trehalose fatty acid ester composition according to Claim 1, wherein
the composition comprises at least two types of esters selected from a group consisting of a pentaester, a hexaester, a heptaester, and an octaester, and
the sum amount of the esters relative to the total peak area, calculated by high performance liquid chromatography analysis is from 30 to 100% by area.

3. The trehalose fatty acid ester composition according to either one of Claim 1 and Claim 2, wherein:
over 90% by mass of all the fatty acid residues held by all the trehalose fatty acid esters in the composition are a palmitic acid residue,
the hydroxyl value of the trehalose fatty acid ester is from 15 to 110, and
the sum amount of a pentaester, a hexaester, a heptaester, and an octaester, relative to the total peak area, calculated by high performance liquid chromatography analysis is from 80 to 100% by area; or
over 60% by mass of all the fatty acid residues held by all the trehalose fatty acid esters in the composition are a stearic acid residue,
the hydroxyl value is from 15 to 110, and
the sum amount of a pentaester, a hexaester, a heptaester, and an octaester, relative to the total peak area, calculated by high performance liquid chromatography analysis is from 70 to 100% by area.

4. The trehalose fatty acid ester composition according to either one of Claim 1 and Claim 2, wherein:
(1) over 90% by mass of all the fatty acid residues held by all the trehalose fatty acid esters in the composition are a palmitic acid residue,
the hydroxyl value is from 15 to 45,
the sum amount of a hexaester, relative to the total peak area, calculated by high performance liquid chromatography analysis is from 10 to 25% by area, and
the sum amount of a heptaester and an octaester, relative to the total peak area, calculated by high performance liquid chromatography analysis is from 70 to 90% by area;
(2) over 90% by mass of all the fatty acid residues held by all the trehalose fatty acid esters in the composition are a stearic acid residue,
the hydroxyl value is from 15 to 110,
the sum amount of a tetraester, a pentaester, and a hexaester, relative to the total peak area, calculated by high performance liquid chromatography analysis is from 10 to 70% by area, and
the sum amount of a heptaester and an octaester, relative to the total peak area, calculated by high performance liquid chromatography analysis is from 20 to 90% by area; or
(3) over 90% by mass of all the fatty acid residues held by all the trehalose fatty acid esters in the composition are at least one type of residue selected from the group consisting of a palmitic acid residue and a stearic acid residue,
all the trehalose fatty acid esters in the composition have both the palmitic acid residue and the stearic acid residue,
over 50% by mass of all the fatty acid residues are a stearic acid residue,
the hydroxyl value is from 15 to 110,
the sum amount of a tetraester, a pentaester, and a hexaester, relative to the total peak area, calculated by high performance liquid chromatography analysis is from 22 to 70% by area, and
the sum amount of a heptaester and an octaester, relative to the total peak area, calculated by high performance liquid chromatography analysis is from 20 to 90% by area.

5. A wax composition, comprising the trehalose fatty acid ester composition according to any one of Claim 1 to Claim 4.

6. A crystal modifier, comprising the trehalose fatty acid ester composition according to any one of Claim 1 to Claim 4.

7. The crystal modifier according to Claim 6, further comprising a wax.

8. The crystal modifier according to either one of Claim 6 and Claim 7, for use in a solid cosmetic.

9. A solid cosmetic, comprising the trehalose fatty acid ester composition according to any one of Claim 1 to Claim 4.

10. The solid cosmetic according to Claim 9, comprising the trehalose fatty acid ester composition at 0.5 to 30% by mass relative to a total mass of the solid cosmetic.

11. The solid cosmetic according to Claim 10, further comprising a wax.

12. The solid cosmetic according to Claim 11, wherein a sum amount of the trehalose fatty acid ester composition and the wax relative to the total mass of the solid cosmetic is from 1 to 30% by mass.

13. A solid cosmetic, comprising the wax composition according to Claim 5.

14. The solid cosmetic according to Claim 13, wherein the amount of the wax composition relative to the total mass of the solid cosmetic is from 1 to 30% by mass.

15. A solid cosmetic, comprising the crystal modifier according to any one of Claim 6 to Claim 8.
